# EUROPEAN PATENT APPLICATION

(11) **EP 1 329 507 A1**
(43) Date of publication of application: **23.07.2003**
(21) Application number: 01974763.3
(22) Date of filing: 10.10.2001
(51) Int. Cl.: C12N 15/11, C07K 14/00, C07K 16/00, C12P 21/08, C12N 5/10, A61K 35/74, A61K 31/711, A23L 1/30

(54) **ARTIFICIAL PROTEIN HAVING POTENTIATED IMMUNOGENICITY OF EPITOPE**

(30) Priority: 13.10.2000 JP 2000314288
(71) Applicant: JAPAN SCIENCE AND TECHNOLOGY CORPORATION, Kawaguchi-shi, Saitama 332-0012 (JP)
(72) Inventor: SHIBA, Kiyotaka, Toshima-ku, Tokyo 170-0012 (JP); OHNO, Tsuneya, Setagaya-ku, Tokyo 158-0081 (JP)
(74) Representative: Harding, Charles Thomas
(86) International application number: JP0108893
(87) International publication number: WO02033074

(57) **Abstract**

The present invention provides a method for efficiently constructing a specific antibody having a neutralizing activity for AIDS or the like, by imparting an ability to induce a strong immunoresponse to peptidic epitope such as the gp120 loop 3 of HIV or the like, from which sufficient immunoresponsive induction activity cannot be obtained by the conventional methods. An artificial protein having epitope with potentiated immunogenicity, comprised of an amino acid sequence containing whole or part of an amino acid sequence of a peptidic epitope and having a property imparted thereto of assisting the formation of the high-order structure of protein, and an amino acid sequence having a property imparted thereto of assisting the antigen presentation treatment caused by immunocompetent cell, is prepared. Subsequently, an antibody against the above-mentioned peptidic epitope is efficiently constructed by a conventional method with the use of said artificial protein.

## Description

### Technical Field

The present invention relates to an artificial protein having epitope with potentiated immunogenicity, a derivative agent for immunoresponse comprising said artificial protein, a method for producing an antibody that uses said artificial protein as an antigen, a functional food comprising said artificial protein as an active ingredient, an agent inducing desensitive and immunologically tolerant state comprised of enterobacteria that express said artificial protein, a DNA vaccine for inducing immunoresponse comprising a DNA that encodes said artificial protein, or the like.

### Background Art

With the emergence of molecular evolution engineering, proteins or genetic DNAs that encode them, which form the essential part of bioreaction, are being created artificially in the laboratories. This technology has made it possible to produce enzymes and proteins having novel activities that do not exist in nature, and proteins having structures that greatly differ from that of natural proteins, and their various applications to the medical and engineering fields are expected. In molecular evolution engineering, a method is conducted wherein a molecule having the activity of the interest is selected from a random pool of polymers with block units of amino acids or nucleotides that construct proteins or genes that encode them. For example, the group of Szostak et al. constructed a DNA group having a random 'sequence with a length of 100 basis by using a DNA synthesizer, the DNA group was transcribed into RNA in vitro, an RNA group with a diversity of 10¹³ was prepared, RNA molecules that bind specifically to a specific pigment were selected from the RNA group, and reported which RNA molecule having which sequence structure satisfies the given function (Nature, 346: 818-822, 1990). With the use of the same approach, the group of Szostak et al. have further succeeded in creating an RNA molecule having a more complex activity such as ligase activity (Science, 261: 1411-1418, 1993).

Meanwhile, there is a hypothesis that genes might have been born by repetitive polymerization of small genes (Proc. Natl. Acad. Sci. USA, 80: 3391-3395, 1983), and it is considered that a polypeptide abundant in repetitive structure easily form a stable high-order structure. Therefore, in molecular evolution engineering, which covers large proteins and genes, a technique to repeatedly polymerize short structure units to synthesize a macromolecule is demanded (Nature, 367: 323-324, 1994). A rolling circle synthesis method has been reported (Proc. Natl. Acad. Sci. USA, 92: 4641-4645, 1995) as a method to obtain repetitive polymers with short DNA units. However, the reaction system in this method is complex, since many steps such as phosphorylation reaction, ligation reaction, polymerization reaction, double strand formation reaction or the like, have to be carried out.

In this connection, as a method for constructing a repetitive polymer of microgenes efficiently and simply, the present inventors propose a microgene polymerization method (Japanese Laid-Open Patent Publication No. 09-322775), characterized in that polymerization reaction is conducted by action of DNA polymerase to oligonucleotide A and oligonucleotide B wherein at least part of the sequence is complementary to each other.

Further, a DNA that encodes an amino acid polymers having repeating units similar to those of natural proteins such as silk protein, elastin and the like (Japanese Laid-Open Patent Publication No. 10-14586), a gene cassette that encodes an artificial protein having a repetitive amino acid sequence (U.S. Patent No. 5089406), a synthesized repeated DNA for constructing a large polypeptide having repetitive sequences of amino acids (U.S. Patent No. 5641648), a DNA sequence that encodes peptides having repetitive units of amino acids (U.S. Patent No. 5770697) and a synthesized repeated DNA for constructing large polypeptides that include repetitive sequences of amino acids (U.S. Patent No. 5830713), are known. In addition, when a DNA fragment wherein one of the 6 open reading frames easily forms an α helix structure is designed, it has been reported that the library constructed therefrom encodes stable proteins at a high frequency, and in the same manner, that a DNA fragment wherein one of the 6 open reading frames easily forms a β strand structure is designed (Proc. Natl. Acad. Sci. USA, 94: 3805-3810, 1997).

Heretofore, as a method for potentiating the antigenicity of peptidic epitope, the method wherein many peptidic epitopes are tandemly ligated, and the tandem polymer consisting of the various peptidic epitopes obtained is used as an immunogen (J. Immunol. 153: 5634-5642, 1994), is known. For example, the description of U.S. Patent No. 5,951,986 discloses the use of a peptide with a polyvalent HIV epitope as an immunogen. Further, a method of constructing a macromolecule that links ramosely and has a polyvalent epitope (U.S. Patent No. 5,229,490, Published Japanese Translation of a PCT Application No. 08-511007) and a method of using a phage that presents a peptidic epitope as an immunogen (Nature Biotechnology, 18: 873-876, 2000) are also known.

The method for tandemly polymerizing peptidic epitopes as mentioned above is easy, however, it does not always function effectively in all epitopes (Mol. Immunol. 34: 599-608, 1997). For example, in a gp120 loop 3 peptide of HIV, the type which is tandemly polymerized and further fused with a carrier protein shows improvement in the reaction to induce immunity in mice, but in a condition where there is no fusion with carrier proteins, no significant difference with immunity using peptide alone is found (Vaccine 17: 2392-2399, 1999). For such peptidic epitope with extremely weak immunogenicity, the development of a new method that imparts strong immunogenicity has been desired. An object of the present invention is to provide a method to impart the ability to induce a strong immunoresponse, even to a peptidic epitope that cannot obtain sufficient immunoresponsive induction activity by the use of conventional methods. More specifically, the object is to provide an artificial protein having epitope with potentiated immunogenicity, a derivative agent for immunoresponse comprising said artificial protein, a method for producing an antibody that uses said artificial protein as an antigen, a functional food comprising said artificial protein as an active ingredient, an agent inducing desensitive and immunologically tolerant state comprised of enterobacteria that express said artificial protein, a DNA vaccine for inducing immunoresponse comprising a DNA that encodes said artificial protein, or the like.

### Disclosure of the Invention

In order for a peptidic epitope to have sufficient antigenicity, there is a need for the peptidic epitope to be incorporated into an immunocompetent cell, and after having been processed, to be presented efficiently to B cells and T cells by MHC molecules. In order for these "incorporation", "processing" and "presentation by MHC" reactions to proceed efficiently, it is considered that the peptidic epitope should exist in the proximal context to natural proteins. Based on such idea, "an artificial protein having a property similar to a natural protein that has at least one copy of a peptidic epitope" was designed and constructed, and an experiment to examine the immunogenicity of this artificial protein was first implemented.

The region called loop 3 of the gp120 protein that AIDS virus has, is known as a neutralizing epitope, and some of the antibodies against this epitope have a neutralizing activity which, for example, suppresses the proliferation of virus, and becomes an important antibody that can be used for AIDS therapy. It is known that AIDS virus changes the amino acid sequence of the loop 3 region by rapidly changing its gene arrangement, and avoids the attack from this neutralizing antibody. From the viewpoint of the clinical use of neutralizing antibodies, a rapid construction of a new neutralizing antibody against the AIDS virus variants that newly emerge in this way, will be necessary. However, it is also known that it does not lead to a sufficient immune induction even when the gp120 protein itself is used as an immunogen, or when a peptide corresponding to the loop 3 region is used as an immunogen. Currently, enormous amount of time and effort is needed to construct a new neutralizing antibody (Nature 376: 115, 1995). It has been reported that a sufficient immunity against the loop 3 peptide sequence cannot be induced, even with the use of a tandem polymer having an epitope sequence that is conventionally used as an antigen (Vaccine 17: 2392-2399, 1999). The reason why an antibody against the loop 3 region is not generated even with the use of gp120 protein itself as an antigen, is thought to be because the loop 3 peptide is buried in the inner side of gp120 protein. It is considered that the reason why the peptide corresponding to the loop 3 region only has a weak immunogenicity is because the "incorporation", "processing" and "presentation by MHC" reactions caused by immunocompetent cells does not proceed efficiently in this peptide.

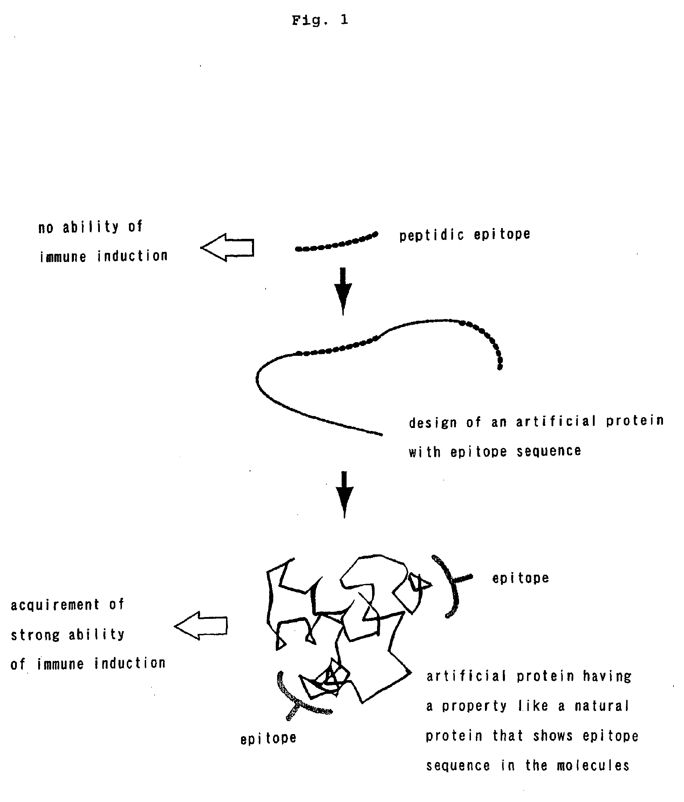

Consequently, an artificial protein was constructed which has at least one copy of a loop 3 peptide sequence on its structure while having a "property similar to a natural protein as a whole", and an efficient immune induction method by using this protein was attempted (Figure 1). For the construction of an artificial protein, methods described in "multifunctional base sequence and artificial gene including the same" (Japanese Patent Application No. 2000-180997) and "method of polymerizing microgene" (Japanese Laid-Open Patent Publication No. 09-322775) were used.

First, a microgene that encodes a loop 3 peptide "RKSIRIQRGPGRTFVTIGKI" known as a neutralizing antigen of AIDS virus, into one of the open reading frames was designed. For example, 6 codons, "CGT", "CGC", "CGA", "CGG", "AGA" and "AGG", correspond to the first R (arginine), and a specific codon was selected from among these. Two codons, "AAA" and "AAG", correspond to the next K (lysine), and a specific codon was selected from among these. When a microgene is designed by sequentially selecting a specific codon by repeating the same procedure, base sequences that encode the above-mentioned neutralizing antigen peptide into one of the open reading frames, will be as many as approximately 1651×10⁸ variants from codon degeneracy. Further, since a single microgene has 3 each of open reading frames in the plus strand and minus strand, it can encode 6 types of different peptides. For example, in 2 different open reading frames in the same direction, 2 peptides that are completely different from the above-mentioned neutralizing antigen peptide will be encoded. A base sequence that encodes a peptide having a property to "easily form a secondary structure" in either of the 2 other open reading frames in the same direction, was searched from among the above-mentioned approximately 1651×10⁸ variants of base sequences by using a calculator.

Sun Enterprise250 of Sun Microsystems, Inc. was used as the calculator, and it was found out to be impossible to simultaneously calculate all of the approximately 1651×10⁸ variants of base sequences. Therefore, calculation was performed for a peptide comprising 13 amino acids, "IRIQRGPGRTFVT", wherein both ends of the above-mentioned neutralizing antigen peptide had been deleted. All the base sequences having the possibility to encode this peptide in one open reading frame was written out in the calculator, and approximately 5×10⁸ variants of base sequences were constructed. The other 2 open reading frames in the same direction of these 5×10⁸ variants of base sequences were translated, and a group of approximately 1506×10⁴ variants of peptide sequences were constructed in the calculator, except for those wherein the translation stopped in the middle due to the emergence of stop codon and the duplicating peptide sequences. Next, peptides having a property to "easily form a secondary structure" were individually calculated from among these approximately 1506×10⁴ variants of peptides, by using a secondary structure prediction program, and were scored. It took more than a week to complete this calculation, but when the results obtained were sorted in descending order of the scores, "ATACGCATTCAGAGAGGCCCTGGCCGCACTTTTGTTACT" was selected as the base sequence that very easily form an α helix at the second open reading frame.

Since the above-mentioned calculation was performed to the 13 amino acid residues in the center portion of 20 amino acid residues of the AIDS virus neutralizing antigen peptide, the same calculation was performed also to both ends that were not calculated. Microgene "design-25" was obtained from these results. This microgene "design-25" encodes a neutralizing antigen sequence in one of its open reading frames, does not have a stop codon in the other 2 open reading frames, and further, encodes a peptide having a property to easily form an α helix in one of the other 2 open reading frames. It can be said that it is a microgene wherein the 2 biological function structures, "AIDS virus neutralizing antigenicity" and "ability of structure formation", are cryptgenic. Next, said microgene "design-25" was polymerized by using the aforementioned method for polymerizing microgene (Japanese Laid-Open Patent Publication No. 09-322775) of the present inventor, and artificial gene libraries comprised of various artificial genes, wherein "neutralizing antigen sequence" and "sequence that easily forms an α helix" are combined intricately, were constructed. Various artificial proteins were expressed in Escherichia coli by using these artificial gene libraries, and among them, artificial proteins having in some parts AIDS virus neutralizing antigen sequences, while supported by α helix structure as a whole, were obtained. Mice were immunized by using said artificial proteins, and it was confirmed that antiserum against the loop 3 region of gp120 protein can be constructed efficiently and that no significant immune induction occurs by immunization using a synthesized peptide that corresponds to the loop 3 region as a control experiment. Thus, the present invention had been completed.

The present invention relates to: an artificial protein having epitope with potentiated immunogenicity, wherein said artificial protein is comprised of an amino acid sequence containing whole or part of an amino acid sequence of a peptidic epitope (claim 1); the artificial protein having epitope with potentiated immunogenicity according to claim 1, wherein said artificial protein is comprised of an amino acid sequence containing whole or part of an amino acid sequence of a peptidic epitope and having a property imparted thereto of assisting the formation of the high-order structure of protein (claim 2); the artificial protein having epitope with potentiated immunogenicity according to claim 1, wherein said artificial protein is comprised of an amino acid sequence containing whole or part of an amino acid sequence of peptidic epitope and having a property imparted thereto of assisting the antigen presentation treatment caused by an immunocompetent cell (claim 3); the artificial protein having epitope with potentiated immunogenicity according to any of claims 1 to 3, wherein a peptide tag or a marker protein is fused (claim 4); the artificial protein having epitope with potentiated immunogenicity according to claim 4, wherein said peptide tag is a polyhistidine residue (claim 5); the artificial protein having epitope with potentiated immunogenicity according to any of claims 1 to 3, wherein the amino acid sequence of peptidic epitope is an amino acid sequence shown in Seq. ID No. 1 (claim 6); and the artificial protein having epitope with potentiated immunogenicity according to any of claims 1 to 4, wherein the amino acid sequence containing whole or part of an amino acid sequence of peptidic epitope is an amino acid sequence shown in any one of Seq. ID Nos. 24 to 47 (claim 7).

Further, the present invention relates to: an agent for inducing immunoresponse wherein said agent comprises the artificial protein having epitope with potentiated immunogenicity according to any of claims 1 to 7 (claim 8); the agent for inducing immunoresponse according to claim 8, wherein said immunoresponse is humoral immunity (claim 9); the agent for inducing immmunoresponse according to claim 8, wherein said immunoresponse is cellular immunity (claim 10); a method for producing antibody, wherein the artificial protein having epitope with potentiated immunogenicity according to any of claims 1 to 7 is used as an antigen (claim 11); the method for producing antibody according to claim 11, wherein said antibody is a monoclonal antibody (claim 12); an antibody that can be obtained by the method for producing antibody according to claim 11 or 12 (claim 13); a cell that produces the antibody according to claim 13 (claim 14); a host cell comprised of an expression system that is capable of expressing the artificial protein having epitope with potentiated immunogenicity according to any of claims 1 to 7 (claim 15); the host cell according to claim 15, wherein said cell is enterobacteria (claim 16); and an agent for inducing desensitive and immunologically tolerant state, wherein said agent is comprised of the enterobacteria according to claim 16 (claim 17).

Still further, the present invention relates to: a DNA that encodes the artificial protein having epitope with potentiated immunogenicity according to any of claims 1 to 7 (claim 18); the DNA according to claim 18, wherein when open reading frames of a base sequence is different, a peptidic epitope is encoded in at least one open reading frame of said base sequence, and a peptide capable of imparting a property of increasing the antigenicity of said peptidic epitope is encoded in other open reading frame (claim 19); a DNA vaccine for immunoresponsive induction, wherein said vaccine is comprised of the DNA according to claim 18 or 19 (claim 20); the DNA vaccine for immunoresponsive induction according to claim 20, wherein said immunoresponse is humoral immunity (claim 21); the DNA vaccine for immunoresponsive induction according to claim 20, wherein said immunoresponse is cellular immunity (claim 22); a functional food comprising the artificial protein having epitope with potentiated immunogenicity according to any of claims 1 to 7 as an active ingredient (claim 23); and the functional food according to claim 23, wherein said food is capable of inducing a desensitive and immunologically tolerant state (claim 24).

### Brief Description of Drawings

Figure 1 is a view explaining that the artificial protein having epitope with potentiated immunogenicity in the present invention acquires strong ability of immune induction.
Figure 2 is a chart indicating an example of a flow of calculating work on the automatic design of microgene.
Figure 3 is a view indicating the microgene comprised of a designed double stranded multifunctional DNA sequence and an amino acid sequence encoded by the microgene.
Figure 4 is a view indicating an example of a DNA that encodes the artificial protein having epitope with potentiated immunogenicity in the present invention.
Figure 5 is a continuation of Figure 4, which is a view indicating an example of a DNA that encodes the artificial protein having epitope with potentiated immunogenicity in the present invention.
Figure 6 is a view indicating an example of the artificial protein having epitope with potentiated immunogenicity in the present invention.
Figure 7 is a continuation of Figure 6, which is a view indicating an example of the artificial protein having epitope with potentiated immunogenicity in the present invention.
Figure 8 is a continuation of Figure 7, which is a view indicating an example of the artificial protein having epitope with potentiated immunogenicity in the present invention.
Figure 9 is a continuation of Figure 8, which is a view indicating an example of the artificial protein having epitope with potentiated immunogenicity in the present invention.
Figure 10 is a graph showing the result by ELISA of the antisera obtained by using the artificial protein having epitope with potentiated immunogenicity in the present invention.
Figure 11 is a graph showing the result by ELISA of the various diluents of the antisera obtained by using the artificial protein having epitope with potentiated immunogenicity in the present invention.
Figure 12 is a graph showing the result by ELISA of the antisera obtained by using other artificial proteins having epitope with potentiated immunogenicity in the present invention.
Figure 13 is a graph showing the result by ELISA of the antisera obtained by using other artificial proteins having epitope with potentiated immunogenicity in the present invention.

### Best Mode of Carrying Out the Invention

There is no particular limitation to the artificial protein having epitope with potentiated immunogenicity in the present invention, as long as it is a protein or peptide comprised of an amino acid sequence containing whole or part of an amino acid sequence of a peptidic epitope. Here, the artificial protein is a protein or peptide that does not exist in nature. It does not include, for example, tandem polymers of known peptidic epitopes such as the cointegrate of a tandem polymer of the gp120 loop 3 peptide of HIV and a carrier protein, described in the aforementioned literature (Vaccine 17: 2392-2399, 1999), and a fusion protein and a fusion peptide of a known peptidic epitope or a tandem polymer of known peptidic epitopes and known carrier proteins. Preferred exemplifications of said artificial protein having epitope with potentiated immunogenicity are: an artificial protein comprised of an amino acid sequence containing whole or part of an amino acid sequence of a peptidic epitope and having a property imparted thereto of assisting the formation of the high-order structure of protein; and an artificial protein comprised of an amino acid sequence containing whole or part of an amino acid sequence of a peptidic epitope and having a property imparted thereto of assisting the antigen presentation treatment caused by immunocompetent cells.

Examples of the property of assisting the formation of the high-order structure of the above-mentioned protein are properties regarding the ability to form an α helix, the ability to form a secondary structure and the ability to improve hydrophobicity. Further, an example of the property of assisting the antigen presentation treatment caused by immunocompetent cells is a property regarding the ability to improve affinity with the MHC molecules of class I and class II. Other various properties that are made similar to natural proteins can also be imparted. An amino acid sequence having these useful properties can be prepared by adjusting the repeatability of amino acid composition and sequence, or by referring to the known sequences that retain said properties. As an amino acid sequence other than the amino acid sequence derived from the amino acid sequence of the epitope according to the artificial protein having epitope with potentiated immunogenicity in the present invention, a peptide sequence having an additional signal of sugar, for example, can be used, aside from the peptide sequence that may impart a property of assisting the formation of the high-order structure of the above-mentioned protein and from the peptide sequence that may impart a property of assisting the antigen presentation treatment caused by immunocompetent cell. With the addition of said peptide sequence, the synthesized natural protein is glycosylated, and can be used as a hybrid artificial protein of protein-sugar.

Specifically, as the artificial protein in the present invention, an amino acid sequence containing whole or part of the peptidic epitope loop 3, which can impart strong immunogenicity to gp120 loop 3 of HIV, a peptidic epitope with very weak immunogenicity, can be given. An example is an artificial peptide or artificial protein that is comprised of an amino acid sequence shown in any one of Seq. ID Nos. 24 to 47. Including said artificial peptides or artificial proteins, the artificial protein having epitope with potentiated immunogenicity in the present invention can be used as a derivative (drug) agent for immunoresponse to induce immunoresponses such as humoral immunity, cellular immunity and the like in vivo.

Examples of a DNA that encodes the artificial protein having epitope with potentiated immunogenicity in the present invention are: when open reading frames of a base sequence is different, DNA encoding a peptidic epitope in at least one open reading frame of said base sequence and encoding a peptide which can impart a property of increasing the antigenicity of said peptidic epitope in other open reading frames; and DNA encoding a peptidic epitope and a peptide which can impart a property of increasing the antigenicity of said peptidic epitope in the same open reading frame. For example, an expression vector incorporated with said DNA can be used as a DNA vaccine for immunoresponsive induction such as humoral immunity, cellular immunity and the like.

Moreover, the present invention relates to the host cell comprised of an expression system that is capable of expressing the artificial protein having epitope with potentiated immunogenicity in the above-mentioned present invention, examples of host cells are: bacterial prokaryotic cells such as Salmonella, Escherichia coli, streptomyces, Bacillus subtilis, Streptococcus, Staphylococcus and the like; fungus cells such as yeast, Aspergillus and the like; insect cells such as drosophila S2, Spodoptera Sf9 and the like; animal cells such as L cell, CHO cell, COS cell, HeLa cell, C127 cell, BALB/c3T3 cell (including variants deficient in dihydrofolate reductase, thymidine kinase and the like), BHK21 cell, HEK293 cell and the like; plant cell and the like. However, enterobacteria such as Salmonella and the like can be favorably exemplified. Since the state of desensitization and immunological tolerance can be induced by administrating the enterobacteria that expresses said artificial protein to the living organism, these enterobacteria can be expected to be used as a agent inducing desensitive and immunologically tolerant state.

Further, the expression system may be any kind of expression system as long as it can express the above-mentioned artificial protein having epitope with potentiated immunogenicity in the present invention in the host cell. Examples of the expression system are expression system derived from chromosome, episome and virus, such as bacterial plasmid-derived, yeast plasmid-derived, papovavirus such as SV40, vaccinia virus, adenovirus, fowlpox virus, pseudorabies virus, retrovirus-derived vector, bacteriophage-derived, transposon-derived and vectors derived from a combination of these, for example, those derived from genetic factors of plasmid and bacteriophage, such as cosmid and phagemid. This expression system may include a regulatory sequence that not only induces expression but also controls the expression. In addition, an expression vector series that shifts the open reading frame and translates can also be used effectively. The DNA that encodes the artificial protein in the present invention and the expression system incorporated with said DNA that encodes the artificial protein can be introduced into a host cell by the methods described in many of the standard laboratory manuals, such as Davis et al. (BASIC METHODS IN MOLECULAR BIOLOGY, 1986) and Sambrook et al. (MOLECULAR CLONING: A LABORATORY MANUAL, 2nd Ed., Cold Spring Harbor Laboratory Press, Cold Spring Harbor, N.Y., 1989). Examples of the method are calcium phosphate transfection, DEAE-dextran-mediated transfection, transvection, microinjection, cationic lipid-mediated transfection, electroporation, transduction, scrape loading, ballistic introduction, infection or the like.

There is no particular limitation to the method of producing the artificial protein in the present invention, as long as it is a method of producing a protein that is conventionally known. For example, it can be obtained by incorporating a DNA that encodes said artificial protein into an expression vector and transforming the host cell, followed by cultivation of said transformed cell, or by method of peptide synthesis. The DNA that encodes the artificial protein mentioned above can be constructed, for example, by using the method of microgene polymerization as described in the Example. The property of the artificial protein that is imparted to the base sequence that encodes an epitope may exist in the same open reading frame of the microgene or in a different open reading frame, however, the method of constructing a DNA that encodes artificial protein is not limited to these. In addition, a peptide tag for separation and purification such as polyhistidine residue and the like can be made to exist in a part of the amino acid sequence of said artificial protein, for example, by making a base sequence encoding a polyhistidine residue exist at the coding region derived from the above-mentioned expression vector.

Further, the artificial protein in the present invention may be used as a fusion protein and fusion peptide ligated with a marker protein and/or peptide tag. There is no particular limitation to the marker protein, as long as it is a marker protein conventionally known. Specific examples of the marker protein are alkaline phosphatase, Fc region of an antibody, HRP, GFP and the like. Specific exemplifications of the peptide tag are those that are conventionally known, such as Myc tag, His tag, FLAG tag, GST tag and the like. Said fusion protein and fusion peptide can be constructed by an ordinary method, and are useful for the purification of the artificial protein in the present invention using the affinity of Ni-NTA and His tag, the detection of proteins having a T cell induction activity, the quantification of antibodies against the artificial protein in the present invention, a diagnostic marker of immunodeficiency syndrome or the like, and also as a laboratory reagent of this field.

There is no particular limitation to the method for producing an antibody in the present invention as long as it is a method that uses the artificial protein having epitope with potentiated immunogenicity as an antigen. Specific examples of the types of said antibody are immunospecific antibodies such as monoclonal antibody, polyclonal antibody, chimeric antibody, single stranded antibody, humanized antibody and the like. These antibodies can be constructed by ordinary methods with the use of the artificial protein having epitope with potentiated immunogenicity in the present invention or a part of it as an antigen. From the point of its specificity, a monoclonal antibody is favorable among them, and particularly, the monoclonal antibody having a neutralizing activity against the proliferation of AIDS virus is more preferable. The antibodies such as said monoclonal antibody and the like are useful, for example, not only for the therapy of immunodeficiency syndrome or the like such as AIDS, but also for the elucidation of the onset mechanism of immunodeficiency syndrome, such as AIDS and the like.

Further, the antibody of the present invention can be produced by administrating the artificial protein having epitope with potentiated immunogenicity in the present invention or a part of it, or a cell expressing a complex comprised of a part of said artificial protein and MHC on the membrane surface, to an animal (preferably non-human), by using the common protocol. For example, in the preparation of a monoclonal antibody, arbitrary methods that generate an antibody produced by the culture of continuous cell line, such as hybridoma method (Nature 256, 495-497, 1975), trioma method, human B cell hybridoma method (Immunology Today 4, 72, 1983) and EBV-hybridoma method (MONOCLONAL ANTIBODIES AND CANCER THERAPY, pp. 77-96, Alan R. Liss, Inc., 1985) can be used.

The method for preparing a single stranded antibody (U.S. Patent No. 4,946,778) can be applied to generate a single stranded antibody against the above-mentioned artificial protein in the present invention. Further, a transgenic mouse or other mammals and the like can be used to express a humanized antibody, a clone that expresses the artificial protein having epitope with potentiated immunogenicity in the present invention can be isolated and identified by the use of the above-mentioned antibody, and its polypeptide can be purified by affinity chromatography. There is a possibility that the antibody against a peptide containing the artificial protein having epitope with potentiated immunogenicity in the present invention and its antigenic epitope, can be used for diagnosis and therapy of immunodeficiency syndromes such as AIDS and the like. Further, an established cultured cell is preferable as the cell that produces the above-mentioned antibody in the present invention. For example, monoclonal antibody-producing hybridoma, B cell line or the like can be exemplified.

The functional food in the present invention may be any kind of food as long as it comprises the artificial protein having epitope with potentiated immunogenicity in the present invention as an active ingredient. Said food can be obtained by using said artificial protein as a part of the food and drink material, or by adding or admixing it during the manufacturing process or after being manufactured. There is no particular limitation to said functional food, and specific examples are: baked goods such as cookie, bread, cake, rice cracker and the like; tablet candy such as lemon pop candy and the like; Japanese confectionery such as a sweet jellied adzuki-bean paste and the like; cold confectionery such as pudding, jelly, ice cream and the like; sweets such as chewing gum, candy and the like; snacks such as cracker, chips and the like; noodles such as wheat noodle, buckwheat noodle and the like; fish cake such as steamed fish paste, ham, fish sausage and the like; dairy product such as cheese, butter and the like; seasoning such as soybean paste, soy sauce, dressing, mayonnaise, sweetener and the like; daily dish such as bean curd, alimentary yam paste, fish boiled in soy sauce, Chinese dumpling, croquette, salad, soup, stew and the like; various beverages such as yogurt, yogurt drink, juice, cow milk, soy milk, alcoholic beverage, coffee, tea, natural leaf tea, oolong tea, sports drink and the like.

The present invention will be explained further and more specifically hereinafter, with reference to Examples, however, the scope of the present invention is not limited to these examples.

### Example 1

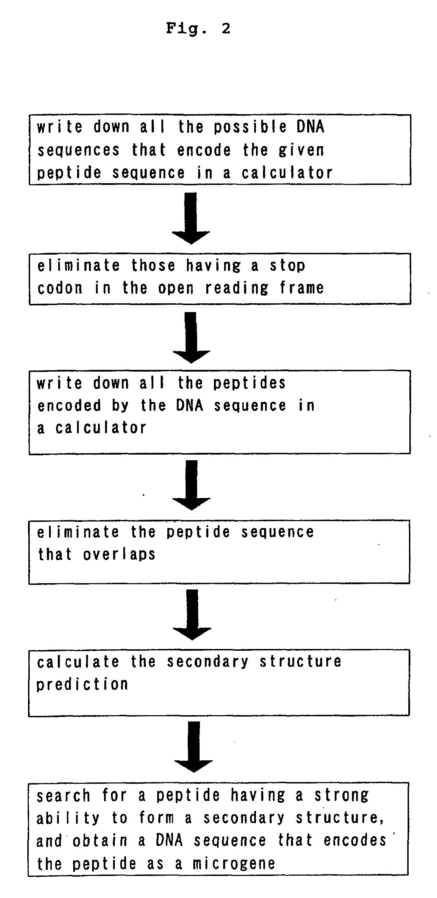

In order to design a microgene wherein a base sequence that encodes a peptide comprising an amino acid sequence shown in Seq. ID No. 1, which is a partial sequence of gp120 protein that the subspecies of a group of HIV virus have, was made to be one of the open reading frames, and wherein the microgene can encode a peptide comprising an amino acid sequence that can easily form a secondary structure in at least either of the other 2 open reading frames in the same direction, a microgene was assembled according to the flow chart shown in Figure 2. Considering the throughput of the calculator, the 20 amino acid residues shown in Seq. ID No. 1 were divided into peptides comprised of 3 amino acid sequences that are partial sequences partly overlapping to each other, that is, 3 amino acid sequences shown in Seq. ID No. 2, Seq. ID No. 3 and Seq. ID No. 4, respectively, and calculation was conducted to each of them.

In the case of a base sequence that encodes a peptide comprised of an amino acid sequence shown in Seq. ID No. 3, approximately 5×10⁸ variants of base sequences, which is the total base sequences (base length 13×3=39) capable of encoding the peptide comprised of the amino acid sequence shown in Seq. ID No. 3 in one of the open reading frames, was constructed in the calculator. From among this sequence group, approximately 1135×10⁴ variants of base sequences that do not have a stop codon at the other 2 open reading frames in the same direction were selected in the calculator. Next, the total amino acid sequences of approximately 227×10⁵ variants, which are encoded by the other 2 open reading frames in the same direction with the open reading frame 1 of these selected base sequences were constructed in the calculator. From among the peptide group comprised of these amino acid sequences, duplicating sequences having the same amino acid sequence were eliminated, and approximately 1506×10⁴ variants of peptide groups each having a different sequence were selected. For each peptide group, its ability to form a secondary structure of α helix and β sheet was determined by a score using the aforementioned secondary structure prediction program. From among the peptides expected to have a high ability of forming a secondary structure, a peptide comprised of an amino acid sequence shown in Seq. ID No. 5 was selected. This peptide is an amino acid sequence encoded in the second open reading frame of the base sequence shown in Seq. ID No. 6, and was the sequence which was expected to easily form an α helix structure.

Calculation in the same manner as described above was conducted also to the base sequences that encode the peptide comprising the amino acid sequence shown in Seq. ID No. 2. A peptide comprising the amino acid sequence shown by Seq. ID No. 7 was selected, which encodes a peptide comprising an amino acid sequence shown by Seq. ID No. 2 in the first open reading frame, and a peptide with high possibility of forming α helix in the second open reading frame where the sequence of 4 to 6 amino acids of the peptide is identical with the sequence of 1 to 3 amino acids in the amino acid sequence shown by Seq. ID No. 5. This peptide is an amino acid sequence that is encoded in the second open reading frame of the base sequence shown in Seq. ID No. 8.

Calculation in the same manner as described above was conducted also to Seq. ID No. 4. A peptide comprising the amino acid sequence shown by Seq. ID No. 9 was selected, which encodes a peptide comprising an amino acid sequence shown by Seq. ID No. 4 in the first open reading frame, and a peptide with high possibility of forming α helix in the second open reading frame where the sequence of 1 to 3 amino acids of the peptide is identical with the sequence of 11 to 13 amino acids in the amino acid sequence shown by Seq. ID No. 5. This peptide is an amino acid sequence that is encoded in the second open reading frame of the base sequence shown in Seq. ID No. 10.

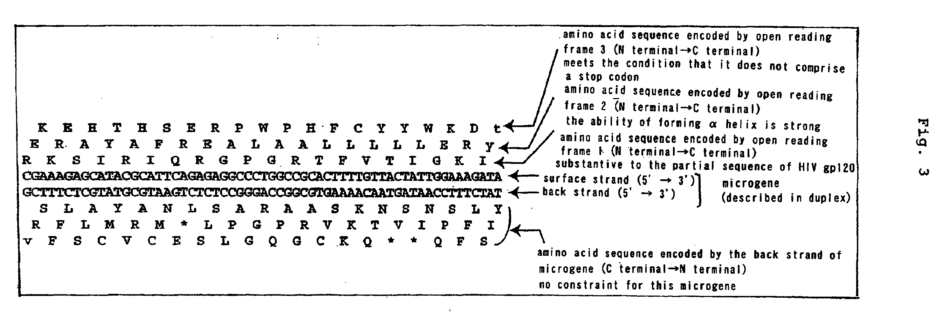

The base sequences shown in each of Seq. ID No. 6, Seq. ID No. 8 and Seq. ID No. 10 that were obtained by the above-mentioned operation, were ligated in consideration of duplication, and a microgene "design-25" having a base sequence shown in Seq. ID No. 11 was obtained. As shown in Figure 3, this designed microgene encodes the partial sequence of gp120 protein, that the subspecies of a group of HIV virus has, in the first open reading frame, and encodes the sequence of a peptide that easily forms α helix structure in the second open reading frame. In the case of microgene design-25, no limitation such as the avoidance of the emergence of a stop codon has been put to the minus strand of the microgene (complementary sequence against the base sequence shown in Seq. ID No. 11).

A microgene polymer library was constructed by using the above-mentioned designed microgene "design-25" as a starting material, and by using the technique of the construction method of a macromolecular microgene polymer described in the aforementioned Japanese Laid-Open Patent Publication No.09-322775. KY-1197 comprised of the base sequence shown in Seq. ID No. 12 was used as oligonucleotide A which is to become the base of polymerization, and KY-1198 comprised of the base sequence shown in Seq. ID No. 13 was used as oligonucleotide B, both after having been synthesized. The 10 resudues on the 3' side of oligonucleotide A comprised of 34 nucleotides and the 10 residues on the 3' side of oligonucleotide B comprised of 36 nucleotides were structured as a complementary sequence to each other, except for the 3' ends.

The condition for polymerization reaction by using the above-mentioned oligonucleotide A and oligonucleotide B in a reaction capacity of 50 µL was as follows:

| | |
|---|---|
| KY-1197 | 20 pmol |
| KY-1198 | 20 pmol |
| KCl | 10 mM |
| (NH₄)₂SO₄ | 10 mM |
| Tris-HCl (pH 8.8) | 10 mM |
| MgSO₄ | 2 mM |
| TritonX-100 | 0.1% |
| 2.5 mM dNTP | 7 µL |

After treating the above-mentioned reactant solution for 10 minutes at 94.degree. C., 5.2 units of DNA polymerase (New England Biolabs, Inc., "Vent_{R}") was added.

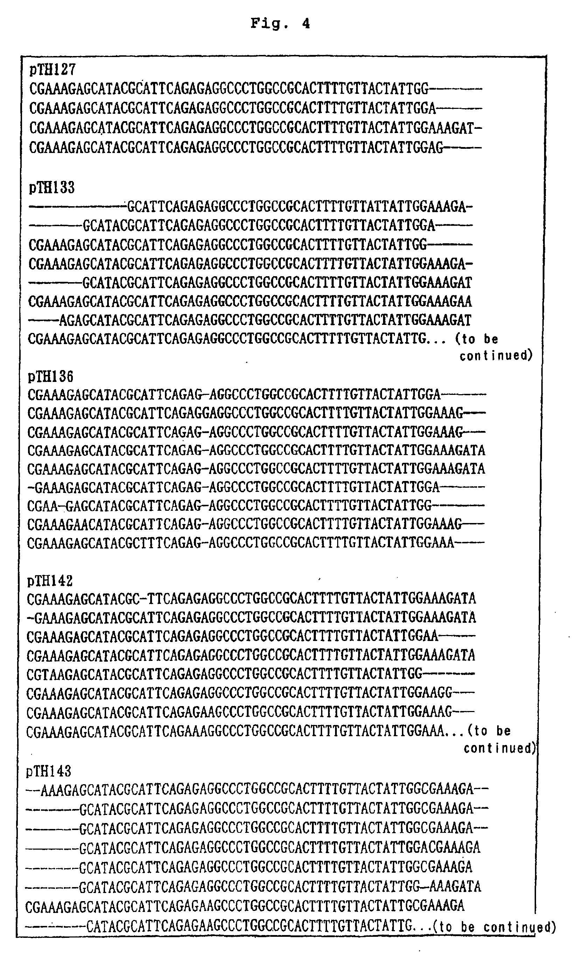

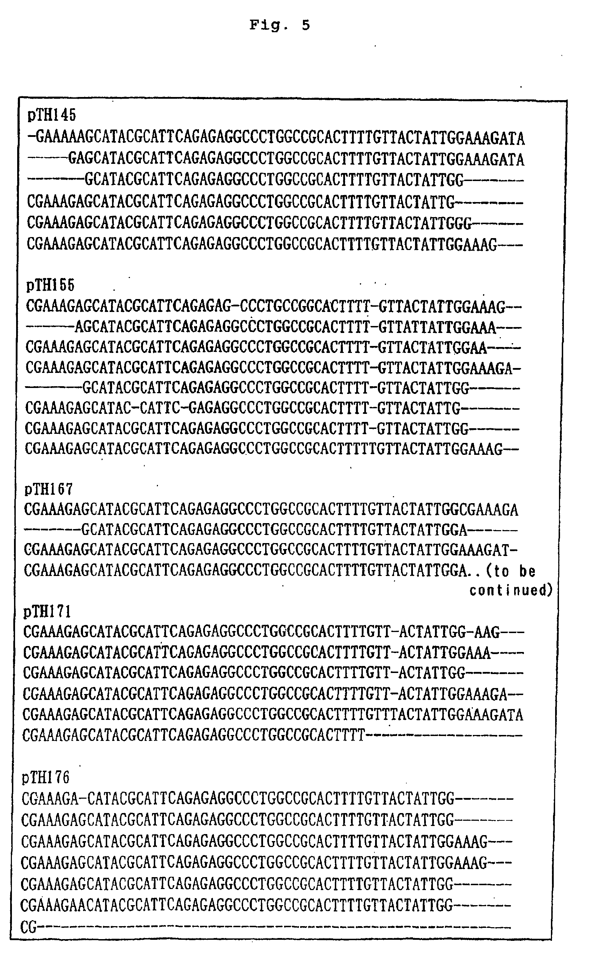

Polymerization reaction was conducted by using the GeneAmp 2400 PCR System of Perkin-Elmer Corporation. As the reaction condition, a repetition of 55 cycles was carried out, wherein the cycle was a thermal denaturation for 10 seconds at 94.degree. C., followed by annealing and stretch reaction for 60 seconds at 66.degree. C., and the last stretch reaction was conducted for 7 minutes at 66.degree. C. The artificial gene in the present invention which was obtained as a polymerization reaction product was cloned in a plasmid vector pTZ19R (Protein Eng., 1:67-74, 1986), and the base sequence of its inserted DNA fragment was determined by using a sequencer (Perkin-Elmer Corporation). The 10 DNA fragments that had been cloned, that is, pTH127, pTH133, pTH136, pTH142, pTH143, pTH145, pTH155, pTH167, pTH171 and pTH176 are shown in Figures 4 and 5. Among these, the inserted fragment of pTH133, pTH142, pTH143 and pTH167 were long, and since they have a property of repetitive sequence, their base sequence of the total length could not be determined, so that their partial sequences are shown in Figures 4 and 5. Each of the inserted base sequences shown in these Figures 4 and 5, are shown in Seq. ID Nos. 14 to 23, respectively.

The 10 inserted DNA fragments that had been cloned in the plasmid vector pTZ19R were excised, and with consideration to such as the direction and open reading frame, they were cloned again in either one of the expression plasmid vector series pKS600-pKS605, which can be expressed by selecting the direction and open reading frame, thereby expressing the artificial gene, that is the above-mentioned microgene polymer, in the Escherichia coli. These 6 types of expression plasmid vectors, pKS600 to pKS605, are modifications of the cloning sites of pQE-9, pQE-10 and pQE-11, which are expression vector series wherein the open reading frames are dislocated one by one, and are sold by Qiagen K. K. These were constructed so that any one of the 6 open reading frames, which are total of 3 open reading frames each of the minus strand and plus strand, can largely express the translation products in the Escherichia coli.

The 24 types of expression plasmid vectors pTH177 to pTH200, wherein the artificial genes had been inserted, that is, pTH177 wherein the inserted sequence of pTH127 is transferred to pKS601, pTH178 wherein the inserted sequence of pTH133 is transferred to pKS601, pTH179 wherein the inserted sequence of pTH136 is transferred to pKS600, pTH180 wherein the inserted sequence of pTH143 is transferred to pKS600, pTH181 wherein the inserted sequence of pTH145 is transferred to pKS601, pTH182 wherein the inserted sequence of pTH155 is transferred to pKS601, pTH183 wherein the inserted sequence of pTH167 is transferred to pKS601, pTH184 wherein the inserted sequence of pTH171 is transferred to pKS601, pTH185 wherein the inserted sequence of pTH176 is transferred to pKS601, pTH186 wherein the inserted sequence of pTH127 is transferred to pKS603, pTH187 wherein the inserted sequence of pTH133 is transferred to pKS603, pTH188 wherein the inserted sequence of pTH142 is transferred to pKS602, pTH189 wherein the inserted sequence of pTH143 is transferred to pKS602, pTH190 wherein the inserted sequence of pTH155 is transferred to pKS603, pTH191 wherein the inserted sequence of pTH167 is transferred to pKS603, pTH192 wherein the inserted sequence of pTH171 is transferred to pKS603, pTH193 wherein the inserted sequence of pTH176 is transferred to pKS603, pTH194 wherein the inserted sequence of pTH127 is transferred to pKS605, pTH195 wherein the inserted sequence of pTH133 is transferred to pKS605, pTH196 wherein the inserted sequence of pTH143 is transferred to pKS604, pTH197 wherein the inserted sequence of pTH155 is transferred to pKS605, pTH198 wherein the inserted sequence of pTH167 is transferred to pKS605, pTH199 wherein the inserted sequence of pTH171 is transferred to pKS605 and pTH200 wherein the inserted sequence of pTH176 is transferred to pKS605, were introduced into an Escherichia coli XL1Blue line, and it was cultured in the presence of IPTG, which is an expression inducing agent, and an artificial protein having whole or part of the loop 3 peptide sequence of HIV gp120 was obtained.

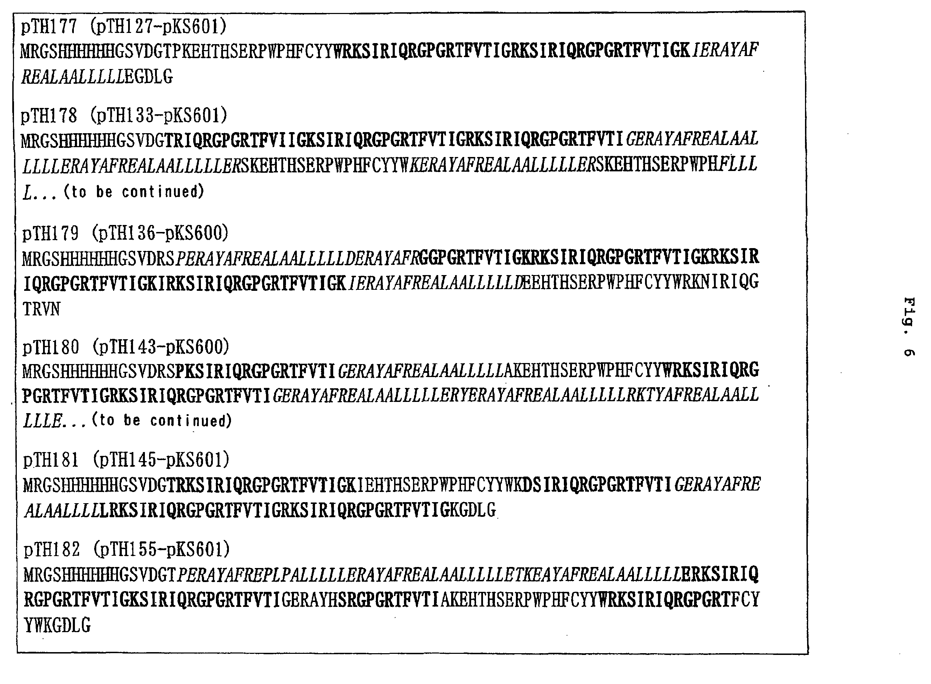

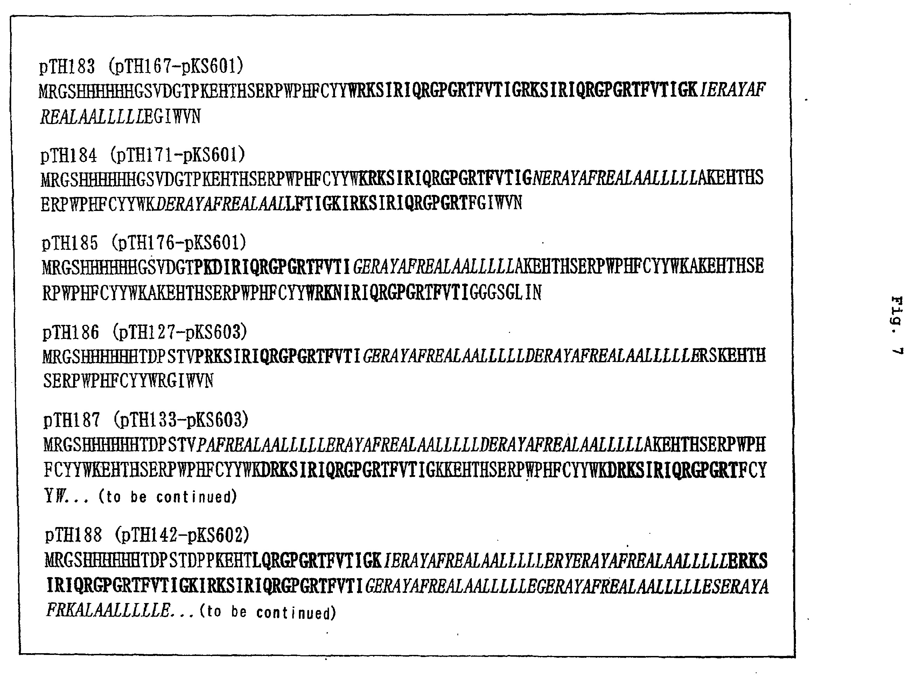

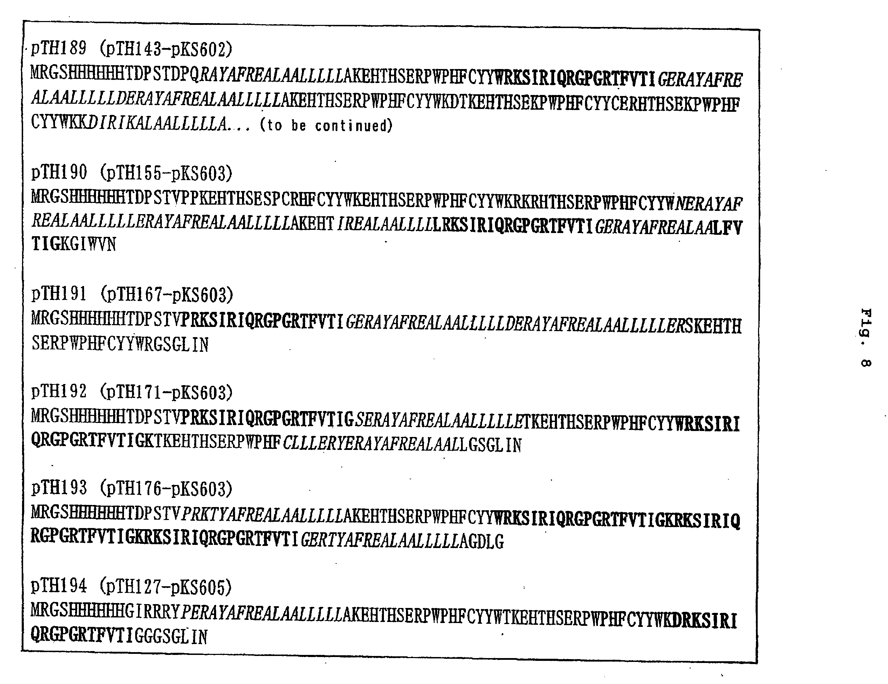

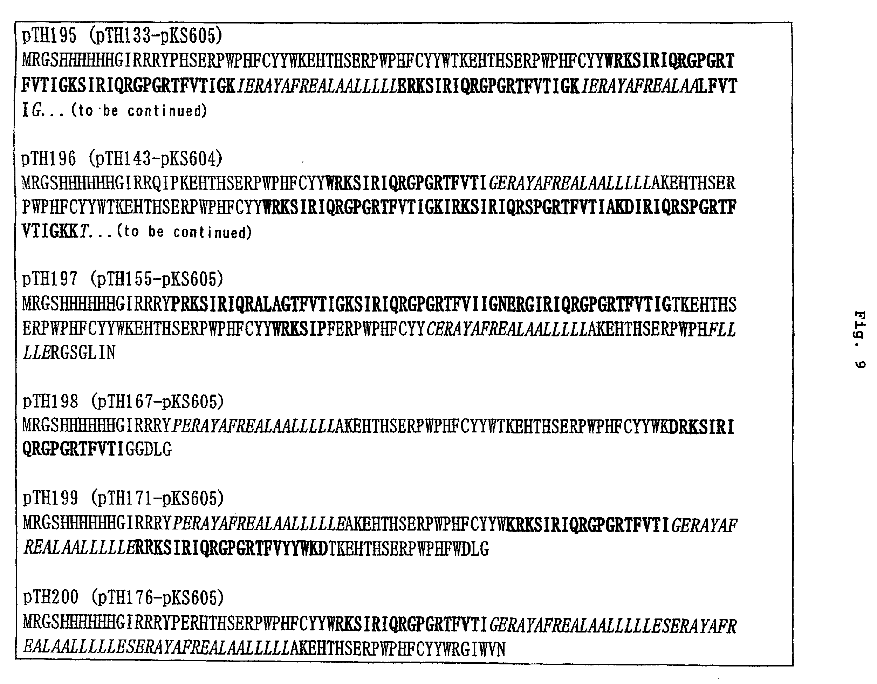

Figures 6 to 9 show the amino acid sequences of the 24 types of artificial proteins that are the translation products of the above-mentioned pTH177 to pTH200, wherein an expression plasmid-derived peptide sequence is fused in their N terminal and C terminal. In Figures 6 to 9, only the peptide sequence of the N terminal region is shown to those where only the partial sequence of the polymer base sequence is known. The inserted peptide sequence of pTH177 shown in Figures 6 to 9 is shown in Seq. ID No. 24, the inserted peptide sequence of pTH178 is shown in Seq. ID No. 25, the inserted peptide sequence of pTH179 is shown in Seq. ID No. 26, the inserted peptide sequence of pTH180 is shown in Seq. ID No. 27, the inserted peptide sequence of pTH181 is shown in Seq. ID No. 28, the inserted peptide sequence of pTH182 is shown in Seq. ID No. 29, the inserted peptide sequence of pTH183 is shown in Seq. ID No. 30, the inserted peptide sequence of pTH184 is shown in Seq. ID No. 31, the inserted peptide sequence of pTH185 is shown in Seq. ID No. 32, the inserted peptide sequence of pTH186 is shown in Seq. ID No. 33, the inserted peptide sequence of pTH187 is shown in Seq. ID No. 34, the inserted peptide sequence of pTH188 is shown in Seq. ID No. 35, the inserted peptide sequence of pTH189 is shown in Seq. ID No. 36, the inserted peptide sequence of pTH190 is shown in Seq. ID No. 37, the inserted peptide sequence of pTH191 is shown in Seq. ID No. 38, the inserted peptide sequence of pTH192 is shown in Seq. ID No. 39, the inserted peptide sequence of pTH193 is shown in Seq. ID No. 40, the inserted peptide sequence of pTH194 is shown in Seq. ID No. 41, the inserted peptide sequence of pTH195 is shown in Seq. ID No. 42, the inserted peptide sequence of pTH196 is shown in Seq. ID No. 43, the inserted peptide sequence of pTH197 is shown in Seq. ID No. 44, the inserted peptide sequence of pTH198 is shown in Seq. ID No. 45, the inserted peptide sequence of pTH199 is shown in Seq. ID No. 46, and the inserted peptide sequence of pTH200 is shown in Seq. ID No. 47.

Among the translation products of the expression vectors pTH177 to pTH200 wherein the above-mentioned 24 types of artificial genes have been inserted, each of the artificial proteins with large amount of expression, that is, pTH177, pTH178, pTH180, pTH181, pTH183, pTH184, pTH185, pTH186, pTH187, pTH188, pTH189, pTH190, pTH192, pTH194, pTH195, pTH196, pTH197, pTH198, pTH199 and pTH200, were purified in the following manner by using a polyhistidine residue of an N terminal derived from an expression vector. Cultivation at 500 ml scale of Escherichia coli XL1Blue line having each of the expression plasmids was started at 37.degree. C. When the OD₆₆₀ of the culture solution reached 0.2, IPTG which is an expression inducing agent was added to make it 0.24 g/l, and was further cultured for 3 hours, the culture solution was centrifuged for 10 minutes at 3,000 g, and the fungus was collected.

The above-mentioned fungus body that had been collected was suspended in 40 ml bacteriolysis buffer (50 mM NaH₂PO₄ (pH 8.0), 10 mM Tris-HCl (pH 8.0), 6 M guanidine hydrochloride, 100 mM NaCl and 1 mM PMSF), incubated for 1 hour at 37.degree. C., and the supernatant obtained by centrifuging at 7,000 g×30 minutes, was mixed with 4 ml of 50% TALON resin (BD Biosciences Clontech) solution which had been pre-treated with bacteriolysis buffer to equilibrate, stirred gently at room temperature for 20 minutes, and then centrifuged at 700 g×5 minutes. Next, the supernatant was thrown away, the precipitate was washed with 20 ml bacteriolysis buffer, stirred gently at room temperature for 10 minutes, centrifuged at 700 g×5 minutes to throw away the supernatant, and washing with the use of bacteriolysis buffer was repeated. Then, 2 ml of bacteriolysis buffer was added therein, suspended with voltex, filled in a column, and after washing with 6 ml washing buffer (50 mM NaH₂PO₄ (pH 7.0), 8 M urea, 100 mM NaCl, 15 mM imidazole), the purified protein that ligated with TALON resin was eluted using an elution buffer (50 mM NaH₂PO₄ (pH 5.0), 20 mM MES (pH 5.0), 8 M urea, 100 mM NaCl, 250 mM imidazole). The eluted fraction containing purified protein was dialized (Pierce Biotechnology, Inc., molecular cutoff 10000) with 50 mM Tris-acetic acid, pH 4.0, 100 mM NaCl, 1 mM EDTA, concentrated with ultrafilter (Amicon Inc., centrip, molecular cutoff 10000), followed by determination of concentration to make a purified sample.

### Example 2

Twenty to 25 µg of the 20 types of purified proteins were injected every 3 weeks for 3 times, into the spleen of a total of 100 mice, that is, 5 mice (BALB/c) for each protein, and were immunized. Five days after final immunization, blood was collected from the retinal blood vessel of the mice. Further, the blood collected from 5 mice (BALB/c) immunized in the same manner using a synthesized peptide of 40 mer including the HIV gp120 loop 3 region, INCTRPNNNTRKSIRIQRGPGRTFNTIGKIGNMRQAHCNI (Seq. ID No. 48), was used as a control (V3). Next, ELISA experiment was conducted with the use of the synthesized peptide of 40 mer including the HIV gp120 loop 3 region by a conventional method, and the ability of immune induction was determined. ELISA was conducted in the following manner. After the above-mentioned synthesized peptide (Seq. ID No. 48) was solid-phased in a 96 well plate (Falcon, 3539), mouse serum was adsorbed as a primary antibody, and after washing, peroxidase conjugated sheep anti-mouse IgG antibody (Amersham Pharmacia Biotech) was added as a secondary antibody. After washing, OPD (o-Phenylenediamine) was colored as a substrate, and the absorbance at wavelength 492 nm was determined.

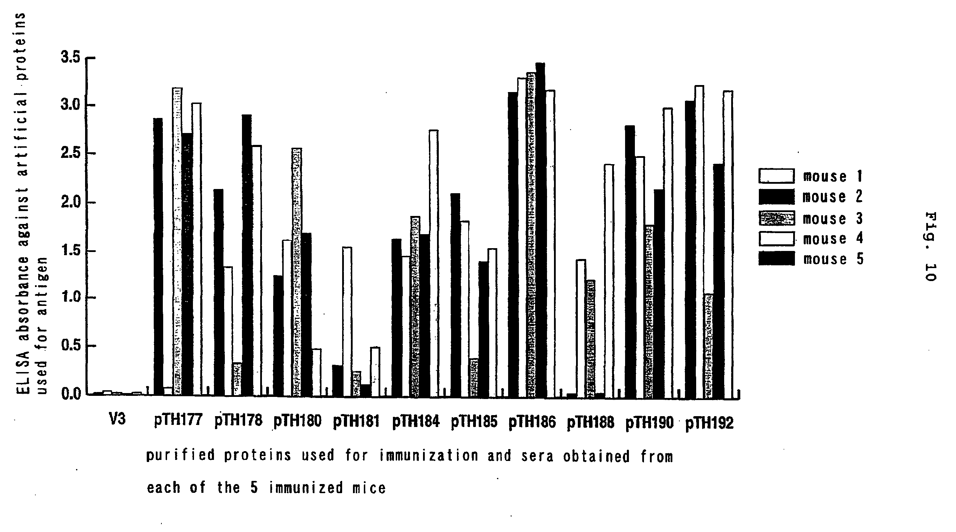

Sera obtained from each of the 5 immunized mice were diluted to 1/2500, in an immunity experiment wherein the purified protein obtained from pTH177, pTH178, pTH180, pTH181, pTH184, pTH185, pTH186, pTH188, pTH190 and pTH192, and the synthesized peptide of 40 mer containing the HIV gp120 loop 3 region as a control, were used as immunogens, and ELISA was conducted. The results are shown in Figure 10. The results shown in Figure 10 indicate that these artificial proteins are recognized as foreign substance in the body of the mice, and induce immunoreaction. Further, from the control experiment wherein mice were immunized with synthesized peptide of 40 mer containing the HIV gp120 loop 3 region, expected results could not be obtained for the antiserum against said synthesized peptide.

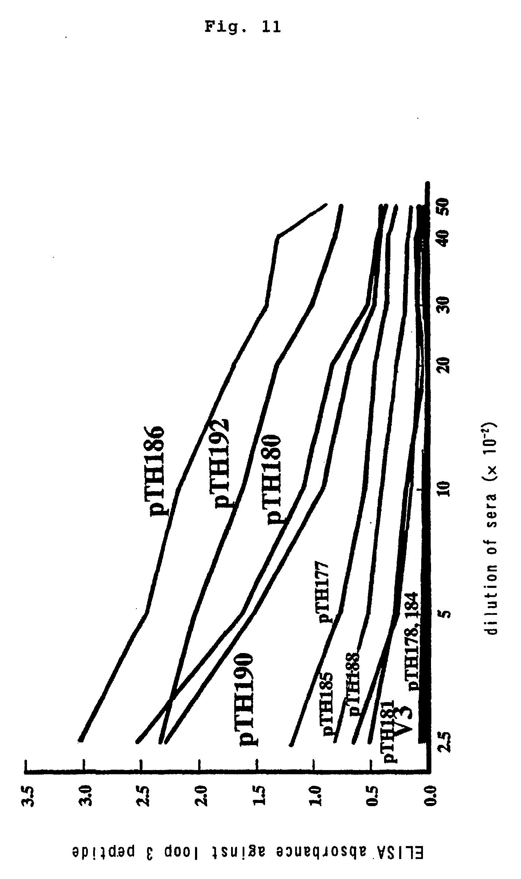

Figure 11 shows the results of ELISA experiment, wherein the same sample used in the experiment in Figure 10 was mixed in the 5 mice having the same antigen, and after treating with different dilutions, the synthesized peptide having a sequence of 40 mer containing the HIV gp120 loop 3 region was made an antigen. This result reveals that an antibody against the part that corresponds to the gp120 loop 3 sequence of the artificial protein is generated in many of the mice.

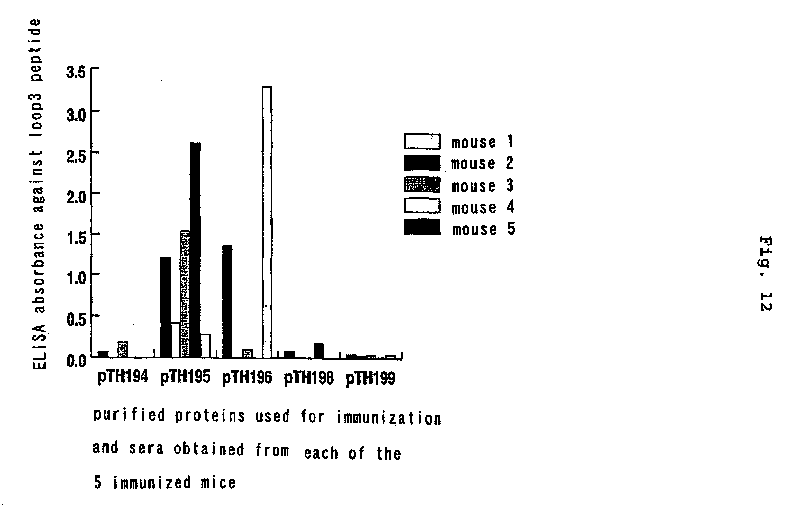

Figure 12 shows the results of ELISA, wherein the sera obtained from each of the 5 immunized mice in an immunity experiment using purified proteins obtained from pTH194, pTH195, pTH196, pTH198 and pTH199, were separately diluted to 1/4000, and the synthesized peptide of 40 mer containing the HIV gp120 loop 3 region was made an antigen. This result reveals that an antibody against the part that corresponds to the gp120 loop 3 sequence of the artificial protein is generated in many of the mice.

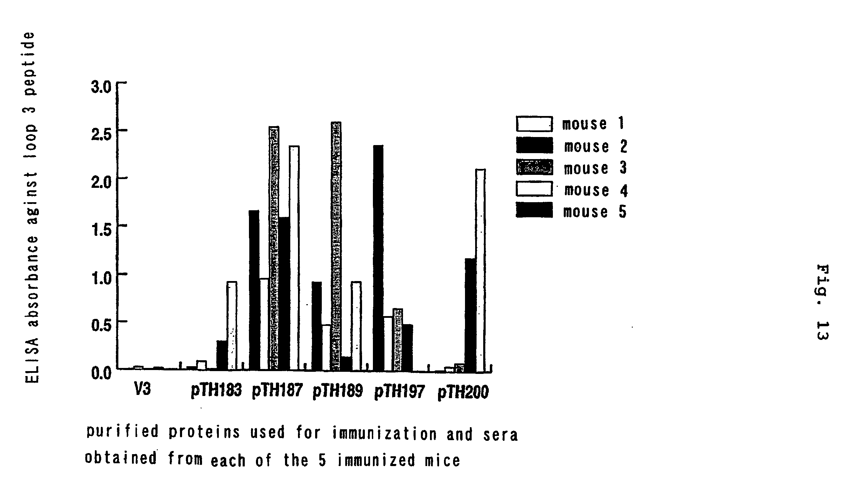

Figure 13 shows the results of ELISA, wherein the sera obtained from each of the 5 immunized mice in an immunity experiment using purified proteins obtained from pTH183, pTH187, pTH189, pTH197 and pTH200, were diluted to 1/5000, and the synthesized peptide of 40 mer containing the HIV gp120 loop 3 region was made an antigen.

### Industrial Applicability

Humoral immunity in vivo can be induced by using the artificial protein having epitope with potentiated immunogenicity in the present invention. Further, an antibody against said epitope can be produced easily and efficiently, by immunizing mice and other animals with said artificial protein. The antibody obtained can be used for therapy and diagnosis that use antibodies. Cellular immunity in vivo can be induced by using the DNA that encodes said artificial protein as a DNA vaccine. It is revealed that the induction of these humoral immunity and cellular immunity can be used as a vaccine against wide range of antigens such as malaria. Further, enterobacteria or the like that express artificial protein can be used for the induction of desensitization and immunological tolerance, and can also be used as a method for inducing immuinoreaction to proteins in a state of immunotolerance in vivo (cancer antigen, fetal antigen or the like).

## Claims

1. An artificial protein having epitope with potentiated immunogenicity, wherein said artificial protein is comprised of an amino acid sequence containing whole or part of an amino acid sequence of a peptidic epitope.

2. The artificial protein having epitope with potentiated immunogenicity according to claim 1, wherein said artificial protein is comprised of an amino acid sequence containing whole or part of an amino acid sequence of a peptidic epitope and having a property imparted thereto of assisting the formation of the high-order structure of protein.

3. The artificial protein having epitope with potentiated immunogenicity according to claim 1, wherein said artificial protein is comprised of an amino acid sequence containing whole or part of an amino acid sequence of peptidic epitope and having a property imparted thereto of assisting the antigen presentation treatment caused by an immunocompetent cell.

4. The artificial protein having epitope with potentiated immunogenicity according to any of claims 1 to 3, wherein a peptide tag or a marker protein is fused.

5. The artificial protein having epitope with potentiated immunogenicity according to claim 4, wherein the peptide tag is a polyhistidine residue.

6. The artificial protein having epitope with potentiated immunogenicity according to any of claims 1 to 3, wherein the amino acid sequence of peptidic epitope is an amino acid sequence shown in Seq. ID No. 1.

7. The artificial protein having epitope with potentiated immunogenicity according to any of claims 1 to 4, wherein the amino acid sequence containing whole or part of an amino acid sequence of peptidic epitope is an amino acid sequence shown in any one of Seq. ID Nos. 24 to 47.

8. An agent for inducing immunoresponse wherein said agent comprises the artificial protein having epitope with potentiated immunogenicity according to any of claims 1 to 7.

9. The agent for inducing immunoresponse according to claim 8, wherein said immunoresponse is humoral immunity.

10. The agent for inducing immmunoresponse according to claim 8, wherein said immunoresponse is cellular immunity.

11. A method for producing antibody, wherein the artificial protein having epitope with potentiated immunogenicity according to any of claims 1 to 7 is used as an antigen.

12. The method for producing antibody according to claim 11, wherein said antibody is a monoclonal antibody.

13. An antibody that can be obtained by the method for producing antibody according to claim 11 or 12.

14. A cell that produces the antibody according to claim 13.

15. A host cell comprised of an expression system that is capable of expressing the artificial protein having epitope with potentiated immunogenicity according to any of claims 1 to 7.

16. The host cell according to claim 15, wherein said cell is enterobacteria.

17. An agent for inducing desensitive and immunologically tolerant state, wherein said agent is comprised of the enterobacteria according to claim 16.

18. A DNA that encodes the artificial protein having epitope with potentiated immunogenicity according to any of claims 1 to 7.

19. The DNA according to claim 18, wherein when open reading frames of a base sequence is different, a peptidic epitope is encoded in at least one open reading frame of said base sequence, and a peptide capable of imparting a property of increasing the antigenicity of said peptidic epitope is encoded in other open reading frame.

20. A DNA vaccine for immunoresponsive induction, wherein said vaccine is comprised of the DNA according to claim 18 or 19.

21. The DNA vaccine for immunoresponsive induction according to claim 20, wherein said immunoresponse is humoral immunity.

22. The DNA vaccine for immunoresponsive induction according to claim 20, wherein said immunoresponse is cellular immunity.

23. A functional food comprising the artificial protein having epitope with potentiated immunogenicity according to any of claims 1 to 7 as an active ingredient.

24. The functional food according to claim 23, wherein said food is capable of inducing a desensitive and immunologically tolerant state.


SEQUENCE LISTING

<110> JAPAN SCIENCE AND TECHNOLOGY CORPORATION

<120> Artificial proteins with enriched immunogen of epitope

<130> YG13-11PCT

<140>
<141>

<150> JP 2000/314288
<151> 2000-10-13

<160> 48

<170> PatentIn Ver. 2.1

<210> 1
<211> 20
<212> PRT
<213> Artificial Sequence

<220>
<223> Description of Artificial Sequence:Designed
      Peptide

<400> 1
Arg Lys Ser Ile Arg Ile Gln Arg Gly Pro Gly Arg Thr Phe Val Thr
  1               5                  10                  15

Ile Gly Lys Ile
             20

<210> 2
<211> 6
<212> PRT
<213> Artificial Sequence

<220>
<223> Description of Artificial Sequence:Designed
      Peptide

<400> 2
Arg Lys Ser Ile Arg Ile

1                   5


<210> 3
<211> 13
<212> PRT
<213> Artificial Sequence

<220>
<223> Description of Artificial Sequence:Designed
      Peptide

<400> 3
Ile Arg Ile Gln Arg Gly Pro Gly Arg Thr Phe Val Thr
  1               5                  10


<210> 4
<211> 7
<212> PRT
<213> Artificial Sequence

<220>
<223> Description of Artificial Sequence:Designed
      Peptide

<400> 4
Phe Val Thr Ile Gly Lys Ile
  1               5


<210> 5
<211> 13
<212> PRT
<213> Artificial Sequence

<220>
<223> Description of Artificial Sequence:Designed
      Peptide

<400> 5
Tyr Ala Phe Arg Glu Ala Leu Ala Ala Leu Leu Leu Leu
  1               5                  10


<210> 6

<211> 39
<212> DNA
<213> Artificial Sequence

<220>
<223> Description of Artificial Sequence:Designed Base
      Sequence

<400> 6
atacgcattc agagaggccc tggccgcact tttgttact                                39


<210> 7
<211> 6
<212> PRT
<213> Artificial Sequence

<220>
<223> Description of Artificial Sequence:Designed
      Peptide

<400> 7
Glu Arg Ala Tyr Ala Phe
  1               5


<210> 8
<211> 18
<212> DNA
<213> Artificial Sequence

<220>
<223> Description of Artificial Sequence:Designed Base
      Sequence

<400> 8
cgaaagagca tacgcatt                                                       18


<210> 9
<211> 7
<212> PRT
<213> Artificial Sequence

<220>
<223> Description of Artificial Sequence:Designed

Peptide

<400> 9
Leu Leu Leu Leu Ala Arg Phe
1               5


<210> 10
<211> 21
<212> DNA
<213> Artificial Sequence

<220>
<223> Description of Artificial Sequence:Designed Base
      Sequence

<400> 10
   tttgttacta ttggcaaaat t                                                21


<210> 11
<211> 60
<212> DNA
<213> Artificial Sequence

<220>
<223> Description of Artificial Sequence:Designed Base
      Sequence

<400> 11
cgaaagagca tacgcattca gagaggccct ggccgcactt ttgttactat tggaaagata 60


<210> 12
<211> 34
<212> DNA
<213> Artificial Sequence

<220>
<223> Description of Artificial Sequence:KY-1197

<400> 12
cgaaagagca tacgcattca gagaggccct ggca                                 34


<210> 13

<211> 36
<212> DNA
<213> Artificial Sequence

<220>
<223> Description of Artificial Sequence:KY-1198

<400> 13
tatctttcca atagtaacaa aagtgcggcc agggca                                36


<210> 14
<211> 221
<212> DNA
<213> Artificial Sequence

<220>
<223> Description of Artificial Sequence:pTH127

<400> 14
cgaaagagca tacgcattca gagaggccct ggccgcactt ttgttactat tggcgaaaga 60
gcatacgcat tcagagaggc cctggccgca cttttgttac tattggacga aagagcatac 120
gcattcagag aggccctggc cgcacttttg ttactattgg aaagatcgaa agagcatacg 180
cattcagaga ggccctggcc gcacttttgt tactattgga g                        221


<210> 15
<211> 422
<212> DNA
<213> Artificial Sequence

<220>
<223> Description of Artificial Sequence:pTH133

<400> 15
gcattcagag aggccctggc cgcacttttg ttattattgg aaagagcata cgcattcaga 60
gaggccctgg ccgcactttt gttactattg gacgaaagag catacgcatt cagagaggcc 120
ctggccgcac ttttgttact attggcgaaa gagcatacgc attcagagag gccctggccg 180
cacttttgtt actattggaa agagcatacg cattcagaga ggccctggcc gcacttttgt 240
tactattgga aagatcgaaa gagcatacgc attcagagag gccctggccg cacttttgtt 300
actattggaa agaaagagca tacgcattca gagaggccct ggccgcactt ttgttactat 360
tggaaagatc gaaagagcat acgcattcag agaggccctg gccgcacttt ttgttactat 420
tg                                                                   422


<210> 16

<211> 507
<212> DNA
<213> Artificial Sequence

<220>
<223> Description of Artificial Sequence:pTH136

<400> 16
cgaaagagca tacgcattca gagaggccct ggccgcactt ttgttactat tggacgaaag 60
agcatacgca ttcagaggag gccctggccg cacttttgtt actattggaa agcgaaagag 120
catacgcatt cagagaggcc ctggccgcac ttttgttact attggaaagc gaaagagcat 180
acgcattcag agaggccctg gccgcacttt tgttactatt ggaaagatac gaaagagcat 240
acgcattcag agaggccctg gccgcacttt tgttactatt ggaaagatag aaagagcata 300
cgcattcaga gaggccctgg ccgcactttt gttactattg gacgaagagc atacgcattc 360
agagaggccc tggccgcact tttgttacta ttggcgaaag aacatacgca ttcagagagg 420
ccctggccgc acttttgtta ctattggaaa gcgaaagagc atacgctttc agagaggccc 480
tggccgcact tttgttacta ttggaaa 507


<210> 17
<211> 456
<212> DNA
<213> Artificial Sequence

<220>
<223> Description of Artificial Sequence:pTH142

<400> 17
cgaaagagca tacgcttcag agaggccctg gccgcacttt tgttactatt ggaaagatag 60
aaagagcata cgcattcaga gaggccctgg ccgcactttt gttactattg gaaagatacg 120
aaagagcata cgcattcaga gaggccctgg ccgcactttt gttactattg gaacgaaaga 180
gcatacgcat tcagagaggc cctggccgca cttttgttac tattggaaag atacgtaaga 240
gcatacgcat tcagagaggc cctggccgca cttttgttac tattggcgaa agagcatacg 300
cattcagaga ggccctggcc gcacttttgt tactattgga aggcgaaaga gcatacgcat 360
tcagagaagc cctggccgca cttttgttac tattggaaag cgaaagagca tacgcattca 420
gaaaggccct ggccgcactt ttgttactat tggaaa 456


<210> 18
<211> 427
<212> DNA
<213> Artificial Sequence

<220>
<223> Description of Artificial Sequence:pTH143

<400> 18
aaagagcata cgcattcaga gaggccctgg ccgcactttt gttactattg gcgaaagagc 60
atacgcattc agagaggccc tggccgcact tttgttacta ttggcgaaag agcatacgca 120
ttcagagagg ccctggccgc acttttgtta ctattggcga aagagcatac gcattcagag 180
aggccctggc cgcacttttg ttactattgg acgaaagagc atacgcattc agagaggccc 240
tggccgcact tttgttacta ttggcgaaag agcatacgca ttcagagagg ccctggccgc 300
acttttgtta ctattggaaa gatacgaaag agcatacgca ttcagagaag ccctggccgc 360
acttttgtta ctattgcgaa agacatacgc attcagagaa gccctggccg cacttttgtt 420
actattg 427

<210> 19
<211> 323
<212> DNA
<213> Artificial Sequence

<220>
<223> Description of Artificial Sequence:pTH145

<400> 19
gaaaaagcat acgcattcag agaggccctg gccgcacttt tgttactatt ggaaagatag 60
agcatacgca ttcagagagg ccctggccgc acttttgtta ctattggaaa gatagcatac 120
gcattcagag aggccctggc cgcacttttg ttactattgg cgaaagagca tacgcattca 180
gagaggccct ggccgcactt ttgttactat tgcgaaagag catacgcatt cagagaggcc 240
ctggccgcac ttttgttact attgggcgaa agagcatacg cattcagaga ggccctggcc 300
gcacttttgt tactattgga aag 323

<210> 20
<211> 426
<212> DNA
<213> Artificial Sequence

<220>
<223> Description of Artificial Sequence:pTH155

<400> 20
cgaaagagca tacgcattca gagagccctg ccggcacttt tgttactatt ggaaagagca 60
tacgcattca gagaggccct ggccgcactt ttgttattat tggaaacgaa agagcatacg 120
cattcagaga ggccctggcc gcacttttgt tactattgga acgaaagagc atacgcattc 180
agagaggccc tggccgcact tttgttacta ttggaaagag catacgcatt cagagaggcc 240
ctggccgcac ttttgttact attggcgaaa gagcatacca ttcgagaggc cctggccgca 300
cttttgttac tattgcgaaa gagcatacgc attcagagag gccctggccg cacttttgtt 360
actattggcg aaagagcata cgcattcaga gaggccctgg ccgcactttt tgttactatt 420
ggaaag 426

<210> 21
<211> 220
<212> DNA
<213> Artificial Sequence

<220>
<223> Description of Artificial Sequence:pTH167

<400> 21
cgaaagagca tacgcattca gagaggccct ggccgcactt ttgttactat tggcgaaaga 60
gcatacgcat tcagagaggc cctggccgca cttttgttac tattggacga aagagcatac 120
gcattcagag aggccctggc cgcacttttg ttactattgg aaagatcgaa agagcatacg 180
cattcagaga ggccctggcc gcacttttgt tactattgga 220

<210> 22
<211> 326
<212> DNA
<213> Artificial Sequence

<220>
<223> Description of Artificial Sequence:pTH171

<400> 22
cgaaagagca tacgcattca gagaggccct ggccgcactt ttgttactat tggaagcgaa 60
agagcatacg cattcagaga ggccctggcc gcacttttgt tactattgga aacgaaagag 120
catacgcatt cagagaggcc ctggccgcac ttttgttact attggcgaaa gagcatacgc 180
attcagagag gccctggccg cacttttgtt actattggaa agacgaaaga gcatacgcat 240
tcagagaggc cctggccgca cttttgttta ctattggaaa gatacgaaag agcatacgca 300
ttcagagagg ccctggccgc actttt 326

<210> 23
<211> 327
<212> DNA
<213> Artificial Sequence

<220>
<223> Description of Artificial Sequence:pTH176

<400> 23
cgaaagacat acgcattcag agaggccctg gccgcacttt tgttactatt ggcgaaagag 60
catacgcatt cagagaggcc ctggccgcac ttttgttact attggcgaaa gagcatacgc 120
attcagagag gccctggccg cacttttgtt actattggaa agcgaaagag catacgcatt 180
cagagaggcc ctggccgcac ttttgttact attggaaagc gaaagagcat acgcattcag 240

agaggccctg gccgcacttt tgttactatt ggcgaaagaa catacgcatt cagagaggcc 300
ctggccgcac ttttgttact attggcg 327

<210> 24
<211> 94
<212> PRT
<213> Artificial Sequence

<220>
<223> Description of Artificial Sequence:pTH177

<400> 24
Met Arg Gly Ser His His His His His His Gly Ser Val Asp Gly Thr
1               5                   10                  15

Pro Lys Glu His Thr His Ser Glu Arg Pro Trp Pro His Phe Cys Tyr
            20                  25                  30

Tyr Trp Arg Lys Ser Ile Arg Ile Gln Arg Gly Pro Gly Arg Thr Phe
        35                  40                  45

Val Thr Ile Gly Arg Lys Ser Ile Arg Ile Gln Arg Gly Pro Gly Arg
    50                  55                  60

Thr Phe Val Thr Ile Gly Lys Ile Glu Arg Ala Tyr Ala Phe Arg Glu
65                  70                  75                  80

Ala Leu Ala Ala Leu Leu Leu Leu Leu Glu Gly Asp Leu Gly
                85                  90

<210> 25
<211> 157
<212> PRT
<213> Artificial Sequence

<220>
<223> Description of Artificial Sequence:pTH178

<400> 25
Met Arg Gly Ser His His His His His His Gly Ser Val Asp Gly Thr
1               5                   10                  15

Arg Ile Gln Arg Gly Pro Gly Arg Thr Phe Val Ile Ile Gly Lys Ser
            20                  25                  30

Ile Arg Ile Gln Arg Gly Pro Gly Arg Thr Phe Val Thr Ile Gly Arg
        35                  40                  45

Lys Ser Ile Arg Ile Gln Arg Gly Pro Gly Arg Thr Phe Val Thr Ile
    50                  55                  60

Gly Glu Arg Ala Tyr Ala Phe Arg Glu Ala Leu Ala Ala Leu Leu Leu
65                  70                  75                  80

Leu Leu Glu Arg Ala Tyr Ala Phe Arg Glu Ala Leu Ala Ala Leu Leu
                85                  90                  95

Leu Leu Leu Glu Arg Ser Lys Glu His Thr His Ser Glu Arg Pro Trp
            100                 105                 110

Pro His Phe Cys Tyr Tyr Trp Lys Glu Arg Ala Tyr Ala Phe Arg Glu
        115                 120                 125

Ala Leu Ala Ala Leu Leu Leu Leu Leu Glu Arg Ser Lys Glu His Thr
    130                 135                 140

His Ser Glu Arg Pro Trp Pro His Phe Leu Leu Leu Leu
145                 150                 155


<210> 26
<211> 160
<212> PRT
<213> Artificial Sequence

<220>
<223> Description of Artificial Sequence:pTH179

<400> 26
Met Arg Gly Ser His His His His His His Gly Ser Val Asp Arg Ser
  1               5                  10                  15

Pro Glu Arg Ala Tyr Ala Phe Arg Glu Ala Leu Ala Ala Leu Leu Leu
                20                  25                  30

Leu Leu Asp Glu Arg Ala Tyr Ala Phe Arg Gly Gly Pro Gly Arg Thr
        35                  40                  45

Phe Val Thr Ile Gly Lys Arg Lys Ser Ile Arg Ile Gln Arg Gly Pro
    50                  55                  60

Gly Arg Thr Phe Val Thr Ile Gly Lys Arg Lys Ser Ile Arg Ile Gln
65                  70                  75                  80

Arg Gly Pro Gly Arg Thr Phe Val Thr Ile Gly Lys Ile Arg Lys Ser
                85                  90                  95

Ile Arg Ile Gln Arg Gly Pro Gly Arg Thr Phe Val Thr Ile Gly Lys
            100                 105                 110

Ile Glu Arg Ala Tyr Ala Phe Arg Glu Ala Leu Ala Ala Leu Leu Leu
        115                 120                 125

Leu Leu Asp Glu Glu His Thr His Ser Glu Arg Pro Trp Pro His Phe
    130                 135                 140

Cys Tyr Tyr Trp Arg Lys Asn Ile Arg Ile Gln Gly Thr Arg Val Asn
145                 150                 155                 160

<210> 27
<211> 160
<212> PRT
<213> Artificial Sequence

<220>
<223> Description of Artificial Sequence:pTH180

<400> 27
Met Arg Gly Ser His His His His His His Gly Ser Val Asp Arg Ser
  1               5                  10                  15

Pro Lys Ser Ile Arg Ile Gln Arg Gly Pro Gly Arg Thr Phe Val Thr
             20                  25                  30

Ile Gly Glu Arg Ala Tyr Ala Phe Arg Glu Ala Leu Ala Ala Leu Leu
         35                  40                  45

Leu Leu Leu Ala Lys Glu His Thr His Ser Glu Arg Pro Trp Pro His
     50                  55                  60

Phe Cys Tyr Tyr Trp Arg Lys Ser Ile Arg Ile Gln Arg Gly Pro Gly
 65                  70                  75                  80

Arg Thr Phe Val Thr Ile Gly Arg Lys Ser Ile Arg Ile Gln Arg Gly
                85                  90                  95

Pro Gly Arg Thr Phe Val Thr Ile Gly Glu Arg Ala Tyr Ala Phe Arg
            100                 105                 110

Glu Ala Leu Ala Ala Leu Leu Leu Leu Leu Glu Arg Tyr Glu Arg Ala
        115                 120                 125

Tyr Ala Phe Arg Glu Ala Leu Ala Ala Leu Leu Leu Leu Leu Arg Lys
    130                 135                 140

Thr Tyr Ala Phe Arg Glu Ala Leu Ala Ala Leu Leu Leu Leu Leu Glu
145                 150                 155                 160

<210> 28
<211> 128
<212> PRT
<213> Artificial Sequence

<220>
<223> Description of Artificial Sequence:pTH181

<400> 28
Met Arg Gly Ser His His His His His His Gly Ser Val Asp Gly Thr
  1               5                  10                  15

Arg Lys Ser Ile Arg Ile Gln Arg Gly Pro Gly Arg Thr Phe Val Thr
             20                  25                  30

Ile Gly Lys Ile Glu His Thr His Ser Glu Arg Pro Trp Pro His Phe
         35                  40                  45

Cys Tyr Tyr Trp Lys Asp Ser Ile Arg Ile Gln Arg Gly Pro Gly Arg
     50                  55                  60

Thr Phe Val Thr Ile Gly Glu Arg Ala Tyr Ala Phe Arg Glu Ala Leu
 65                  70                  75                  80

Ala Ala Leu Leu Leu Leu Leu Arg Lys Ser Ile Arg Ile Gln Arg Gly
                 85                  90                  95

Pro Gly Arg Thr Phe Val Thr Ile Gly Arg Lys Ser Ile Arg Ile Gln
            100                 105                 110

Arg Gly Pro Gly Arg Thr Phe Val Thr Ile Gly Lys Gly Asp Leu Gly
        115                 120                 125

<210> 29
<211> 163
<212> PRT
<213> Artificial Sequence

<220>
<223> Description of Artificial Sequence:pTH182

<400> 29
Met Arg Gly Ser His His His His His His Gly Ser Val Asp Gly Thr
  1               5                  10                  15

Pro Glu Arg Ala Tyr Ala Phe Arg Glu Pro Leu Pro Ala Leu Leu Leu
             20                  25                  30

Leu Leu Glu Arg Ala Tyr Ala Phe Arg Glu Ala Leu Ala Ala Leu Leu
         35                  40                  45

Leu Leu Leu Glu Thr Lys Glu Ala Tyr Ala Phe Arg Glu Ala Leu Ala
     50                  55                  60

Ala Leu Leu Leu Leu Leu Glu Arg Lys Ser Ile Arg Ile Gln Arg Gly
 65                  70                  75                  80

Pro Gly Arg Thr Phe Val Thr Ile Gly Lys Ser Ile Arg Ile Gln Arg
                 85                  90                  95

Gly Pro Gly Arg Thr Phe Val Thr Ile Gly Glu Arg Ala Tyr His Ser
            100                 105                 110

Arg Gly Pro Gly Arg Thr Phe Val Thr Ile Ala Lys Glu His Thr His
        115                 120                 125

Ser Glu Arg Pro Trp Pro His Phe Cys Tyr Tyr Trp Arg Lys Ser Ile
    130                 135                 140

Arg Ile Gln Arg Gly Pro Gly Arg Thr Phe Cys Tyr Tyr Trp Lys Gly
145                 150                 155                 160

Asp Leu Gly

<210> 30
<211> 95
<212> PRT
<213> Artificial Sequence

<220>
<223> Description of Artificial Sequence:pTH183

<400> 30
Met Arg Gly Ser His His His His His His Gly Ser Val Asp Gly Thr
  1               5                  10                  15

Pro Lys Glu His Thr His Ser Glu Arg Pro Trp Pro His Phe Cys Tyr
             20                  25                  30

Tyr Trp Arg Lys Ser Ile Arg Ile Gln Arg Gly Pro Gly Arg Thr Phe
         35                  40                  45

Val Thr Ile Gly Arg Lys Ser Ile Arg Ile Gln Arg Gly Pro Gly Arg
     50                  55                  60

Thr Phe Val Thr Ile Gly Lys Ile Glu Arg Ala Tyr Ala Phe Arg Glu
 65                  70                  75                  80

Ala Leu Ala Ala Leu Leu Leu Leu Leu Glu Gly Ile Trp Val Asn
                 85                  90                  95

<210> 31
<211> 130
<212> PRT
<213> Artificial Sequence

<220>
<223> Description of Artificial Sequence:pTH184

<400> 31
Met Arg Gly Ser His His His His His His Gly Ser Val Asp Gly Thr

1                 5                   10                  15

Pro Lys Glu His Thr His Ser Glu Arg Pro Trp Pro His Phe Cys Tyr
                20                  25                  30

Tyr Trp Lys Arg Lys Ser Ile Arg Ile Gln Arg Gly Pro Gly Arg Thr
            35                  40                  45

Phe Val Thr Ile Gly Asn Glu Arg Ala Tyr Ala Phe Arg Glu Ala Leu
    50                  55                  60

Ala Ala Leu Leu Leu Leu Leu Ala Lys Glu His Thr His Ser Glu Arg
65                  70                  75                  80

Pro Trp Pro His Phe Cys Tyr Tyr Trp Lys Asp Glu Arg Ala Tyr Ala
                    85                  90                  95

Phe Arg Glu Ala Leu Ala Ala Leu Leu Phe Thr Ile Gly Lys Ile Arg
                100                 105                 110

Lys Ser Ile Arg Ile Gln Arg Gly Pro Gly Arg Thr Phe Gly Ile Trp
            115                 120                 125

Val Asn
    130


<210> 32
<211> 132
<212> PRT
<213> Artificial Sequence

<220>
<223> Description of Artificial Sequence:pTH185

<400> 32
Met Arg Gly Ser His His His His His His Gly Ser Val Asp Gly Thr
1                 5                   10                  15

Pro Lys Asp Ile Arg Ile Gln Arg Gly Pro Gly Arg Thr Phe Val Thr
                20                  25                  30

Ile Gly Glu Arg Ala Tyr Ala Phe Arg Glu Ala Leu Ala Ala Leu Leu
            35                  40                  45

Leu Leu Leu Ala Lys Glu His Thr His Ser Glu Arg Pro Trp Pro His

50                  55                  60

Phe Cys Tyr Tyr Trp Lys Ala Lys Glu His Thr His Ser Glu Arg Pro
65                  70                  75                  80

Trp Pro His Phe Cys Tyr Tyr Trp Lys Ala Lys Glu His Thr His Ser
                85                  90                  95

Glu Arg Pro Trp Pro His Phe Cys Tyr Tyr Trp Arg Lys Asn Ile Arg
            100                 105                 110

Ile Gln Arg Gly Pro Gly Arg Thr Phe Val Thr Ile Gly Gly Gly Ser
        115                 120                 125

Gly Leu Ile Asn
    130


<210> 33
<211> 96
<212> PRT
<213> Artificial Sequence

<220>
<223> Description of Artificial Sequence:pTH186

<400> 33
Met Arg Gly Ser His His His His His His Thr Asp Pro Ser Thr Val
 1               5                  10                  15

Pro Arg Lys Ser Ile Arg Ile Gln Arg Gly Pro Gly Arg Thr Phe Val
            20                  25                  30

Thr Ile Gly Glu Arg Ala Tyr Ala Phe Arg Glu Ala Leu Ala Ala Leu
        35                  40                  45

Leu Leu Leu Leu Asp Glu Arg Ala Tyr Ala Phe Arg Glu Ala Leu Ala
    50                  55                  60

Ala Leu Leu Leu Leu Leu Glu Arg Ser Lys Glu His Thr His Ser Glu
65                  70                  75                  80

Arg Pro Trp Pro His Phe Cys Tyr Tyr Trp Arg Gly Ile Trp Val Asn
                85                  90                  95

<210> 34
<211> 158
<212> PRT
<213> Artificial Sequence

<220>
<223> Description of Artificial Sequence:pTH187

<400> 34
Met Arg Gly Ser His His His His His His Thr Asp Pro Ser Thr Val
  1               5                  10                  15

Pro Ala Phe Arg Glu Ala Leu Ala Ala Leu Leu Leu Leu Leu Glu Arg
             20                  25                  30

Ala Tyr Ala Phe Arg Glu Ala Leu Ala Ala Leu Leu Leu Leu Leu Asp
         35                  40                  45

Glu Arg Ala Tyr Ala Phe Arg Glu Ala Leu Ala Ala Leu Leu Leu Leu
     50                  55                  60

Leu Ala Lys Glu His Thr His Ser Glu Arg Pro Trp Pro His Phe Cys
 65                  70                  75                  80

Tyr Tyr Trp Lys Glu His Thr His Ser Glu Arg Pro Trp Pro His Phe
                 85                  90                  95

Cys Tyr Tyr Trp Lys Asp Arg Lys Ser Ile Arg Ile Gln Arg Gly Pro
            100                 105                 110

Gly Arg Thr Phe Val Thr Ile Gly Lys Lys Glu His Thr His Ser Glu
        115                 120                 125

Arg Pro Trp Pro His Phe Cys Tyr Tyr Trp Lys Asp Arg Lys Ser Ile
    130                 135                 140

Arg Ile Gln Arg Gly Pro Gly Arg Thr Phe Cys Tyr Tyr Trp
145                 150                 155


<210> 35
<211> 169
<212> PRT

<213> Artificial Sequence

<220>
<223> Description of Artificial Sequence:pTH188

<400> 35
Met Arg Gly Ser His His His His His His Thr Asp Pro Ser Thr Asp
 1               5                  10                  15

Pro Pro Lys Glu His Thr Leu Gln Arg Gly Pro Gly Arg Thr Phe Val
            20                  25                  30

Thr Ile Gly Lys Ile Glu Arg Ala Tyr Ala Phe Arg Glu Ala Leu Ala
        35                  40                  45

Ala Leu Leu Leu Leu Leu Glu Arg Tyr Glu Arg Ala Tyr Ala Phe Arg
    50                  55                  60

Glu Ala Leu Ala Ala Leu Leu Leu Leu Leu Glu Arg Lys Ser Ile Arg
65                  70                  75                  80

Ile Gln Arg Gly Pro Gly Arg Thr Phe Val Thr Ile Gly Lys Ile Arg
                85                  90                  95

Lys Ser Ile Arg Ile Gln Arg Gly Pro Gly Arg Thr Phe Val Thr Ile
            100                 105                 110

Gly Glu Arg Ala Tyr Ala Phe Arg Glu Ala Leu Ala Ala Leu Leu Leu
        115                 120                 125

Leu Leu Glu Gly Glu Arg Ala Tyr Ala Phe Arg Glu Ala Leu Ala Ala
    130                 135                 140

Leu Leu Leu Leu Leu Glu Ser Glu Arg Ala Tyr Ala Phe Arg Lys Ala
145                 150                 155                 160

Leu Ala Ala Leu Leu Leu Leu Leu Glu
                165


<210> 36
<211> 177
<212> PRT
<213> Artificial Sequence

<220>

<223> Description of Artificial Sequence:pTH189

<400> 36
Met Arg Gly Ser His His His His His His Thr Asp Pro Ser Thr Asp
1               5                   10                  15

Pro Gln Arg Ala Tyr Ala Phe Arg Glu Ala Leu Ala Ala Leu Leu Leu
            20                  25                  30

Leu Leu Ala Lys Glu His Thr His Ser Glu Arg Pro Trp Pro His Phe
        35                  40                  45

Cys Tyr Tyr Trp Arg Lys Ser Ile Arg Ile Gln Arg Gly Pro Gly Arg
    50                  55                  60

Thr Phe Val Thr Ile Gly Glu Arg Ala Tyr Ala Phe Arg Glu Ala Leu
65                  70                  75                  80

Ala Ala Leu Leu Leu Leu Leu Asp Glu Arg Ala Tyr Ala Phe Arg Glu
                85                  90                  95

Ala Leu Ala Ala Leu Leu Leu Leu Leu Ala Lys Glu His Thr His Ser
            100                 105                 110

Glu Arg Pro Trp Pro His Phe Cys Tyr Tyr Trp Lys Asp Thr Lys Glu
        115                 120                 125

His Thr His Ser Glu Lys Pro Trp Pro His Phe Cys Tyr Tyr Cys Glu
    130                 135                 140

Arg His Thr His Ser Glu Lys Pro Trp Pro His Phe Cys Tyr Tyr Trp
145                 150                 155                 160

Lys Lys Asp Ile Arg Ile Lys Ala Leu Ala Ala Leu Leu Leu Leu Leu
                165                 170                 175

Ala


<210> 37
<211> 165
<212> PRT
<213> Artificial Sequence

<220>

<223> Description of Artificial Sequence:pTH190

<400> 37
Met Arg Gly Ser His His His His His His Thr Asp Pro Ser Thr Val
1               5                   10                  15

Pro Pro Lys Glu His Thr His Ser Glu Ser Pro Cys Arg His Phe Cys
            20                  25                  30

Tyr Tyr Trp Lys Glu His Thr His Ser Glu Arg Pro Trp Pro His Phe
        35                  40                  45

Cys Tyr Tyr Trp Lys Arg Lys Arg His Thr His Ser Glu Arg Pro Trp
    50                  55                  60

Pro His Phe Cys Tyr Tyr Trp Asn Glu Arg Ala Tyr Ala Phe Arg Glu
65                  70                  75                  80

Ala Leu Ala Ala Leu Leu Leu Leu Leu Glu Arg Ala Tyr Ala Phe Arg
                85                  90                  95

Glu Ala Leu Ala Ala Leu Leu Leu Leu Leu Ala Lys Glu His Thr Ile
            100                 105                 110

Arg Glu Ala Leu Ala Ala Leu Leu Leu Leu Leu Arg Lys Ser Ile Arg
        115                 120                 125

Ile Gln Arg Gly Pro Gly Arg Thr Phe Val Thr Ile Gly Glu Arg Ala
    130                 135                 140

Tyr Ala Phe Arg Glu Ala Leu Ala Ala Leu Phe Val Thr Ile Gly Lys
145                 150                 155                 160

Gly Ile Trp Val Asn
                165


<210> 38
<211> 97
<212> PRT
<213> Artificial Sequence

<220>
<223> Description of Artificial Sequence:pTH191

<400> 38

Met Arg Gly Ser His His His His His His Thr Asp Pro Ser Thr Val
1               5                   10                  15

Pro Arg Lys Ser Ile Arg Ile Gln Arg Gly Pro Gly Arg Thr Phe Val
            20                  25                  30

Thr Ile Gly Glu Arg Ala Tyr Ala Phe Arg Glu Ala Leu Ala Ala Leu
        35                  40                  45

Leu Leu Leu Leu Asp Glu Arg Ala Tyr Ala Phe Arg Glu Ala Leu Ala
    50                  55                  60

Ala Leu Leu Leu Leu Leu Glu Arg Ser Lys Glu His Thr His Ser Glu
65                  70                  75                  80

Arg Pro Trp Pro His Phe Cys Tyr Tyr Trp Arg Gly Ser Gly Leu Ile
                85                  90                  95

Asn

<210> 39
<211> 132
<212> PRT
<213> Artificial Sequence

<220>
<223> Description of Artificial Sequence:pTH192

<400> 39
Met Arg Gly Ser His His His His His His Thr Asp Pro Ser Thr Val
1               5                   10                  15

Pro Arg Lys Ser Ile Arg Ile Gln Arg Gly Pro Gly Arg Thr Phe Val
            20                  25                  30

Thr Ile Gly Ser Glu Arg Ala Tyr Ala Phe Arg Glu Ala Leu Ala Ala
        35                  40                  45

Leu Leu Leu Leu Leu Glu Thr Lys Glu His Thr His Ser Glu Arg Pro
    50                  55                  60

Trp Pro His Phe Cys Tyr Tyr Trp Arg Lys Ser Ile Arg Ile Gln Arg
65                  70                  75                  80

Gly Pro Gly Arg Thr Phe Val Thr Ile Gly Lys Thr Lys Glu His Thr
                85                  90                  95

His Ser Glu Arg Pro Trp Pro His Phe Cys Leu Leu Leu Glu Arg Tyr
            100                 105                 110

Glu Arg Ala Tyr Ala Phe Arg Glu Ala Leu Ala Ala Leu Leu Gly Ser
        115                 120                 125

Gly Leu Ile Asn
    130


<210> 40
<211> 130
<212> PRT
<213> Artificial Sequence

<220>
<223> Description of Artificial Sequence:pTH193

<400> 40
Met Arg Gly Ser His His His His His His Thr Asp Pro Ser Thr Val
  1               5                  10                  15

Pro Arg Lys Thr Tyr Ala Phe Arg Glu Ala Leu Ala Ala Leu Leu Leu
            20                  25                  30

Leu Leu Ala Lys Glu His Thr His Ser Glu Arg Pro Trp Pro His Phe
        35                  40                  45

Cys Tyr Tyr Trp Arg Lys Ser Ile Arg Ile Gln Arg Gly Pro Gly Arg
    50                  55                  60

Thr Phe Val Thr Ile Gly Lys Arg Lys Ser Ile Arg Ile Gln Arg Gly
 65                  70                  75                  80

Pro Gly Arg Thr Phe Val Thr Ile Gly Lys Arg Lys Ser Ile Arg Ile
                85                  90                  95

Gln Arg Gly Pro Gly Arg Thr Phe Val Thr Ile Gly Glu Arg Thr Tyr
            100                 105                 110

Ala Phe Arg Glu Ala Leu Ala Ala Leu Leu Leu Leu Leu Ala Gly Asp
        115                 120                 125

Leu Gly
    130

<210> 41
<211> 97
<212> PRT
<213> Artificial Sequence

<220>
<223> Description of Artificial Sequence:pTH194

<400> 41
Met Arg Gly Ser His His His His His His Gly Ile Arg Arg Arg Tyr
  1               5                  10                  15

Pro Glu Arg Ala Tyr Ala Phe Arg Glu Ala Leu Ala Ala Leu Leu Leu
             20                  25                  30

Leu Leu Ala Lys Glu His Thr His Ser Glu Arg Pro Trp Pro His Phe
         35                  40                  45

Cys Tyr Tyr Trp Thr Lys Glu His Thr His Ser Glu Arg Pro Trp Pro
     50                  55                  60

His Phe Cys Tyr Tyr Trp Lys Asp Arg Lys Ser Ile Arg Ile Gln Arg
 65                  70                  75                  80

Gly Pro Gly Arg Thr Phe Val Thr Ile Gly Gly Gly Ser Gly Leu Ile
                 85                  90                  95

Asn

<210> 42
<211> 158
<212> PRT
<213> Artificial Sequence

<220>
<223> Description of Artificial Sequence:pTH195

<400> 42
Met Arg Gly Ser His His His His His His Gly Ile Arg Arg Arg Tyr
  1               5                  10                  15

Pro His Ser Glu Arg Pro Trp Pro His Phe Cys Tyr Tyr Trp Lys Glu
            20                  25                  30

His Thr His Ser Glu Arg Pro Trp Pro His Phe Cys Tyr Tyr Trp Thr
        35                  40                  45

Lys Glu His Thr His Ser Glu Arg Pro Trp Pro His Phe Cys Tyr Tyr
    50                  55                  60

Trp Arg Lys Ser Ile Arg Ile Gln Arg Gly Pro Gly Arg Thr Phe Val
65                  70                  75                  80

Thr Ile Gly Lys Ser Ile Arg Ile Gln Arg Gly Pro Gly Arg Thr Phe
                85                  90                  95

Val Thr Ile Gly Lys Ile Glu Arg Ala Tyr Ala Phe Arg Glu Ala Leu
            100                 105                 110

Ala Ala Leu Leu Leu Leu Leu Glu Arg Lys Ser Ile Arg Ile Gln Arg
        115                 120                 125

Gly Pro Gly Arg Thr Phe Val Thr Ile Gly Lys Ile Glu Arg Ala Tyr
    130                 135                 140

Ala Phe Arg Glu Ala Leu Ala Ala Leu Phe Val Thr Ile Gly
145                 150                 155

<210> 43
<211> 163
<212> PRT
<213> Artificial Sequence

<220>
<223> Description of Artificial Sequence:pTH196

<400> 43
Met Arg Gly Ser His His His His His His Gly Ile Arg Arg Gln Ile
1               5                   10                  15

Pro Lys Glu His Thr His Ser Glu Arg Pro Trp Pro His Phe Cys Tyr
            20                  25                  30

Tyr Trp Arg Lys Ser Ile Arg Ile Gln Arg Gly Pro Gly Arg Thr Phe
        35                  40                  45

Val Thr Ile Gly Glu Arg Ala Tyr Ala Phe Arg Glu Ala Leu Ala Ala
    50                  55                  60

Leu Leu Leu Leu Leu Ala Lys Glu His Thr His Ser Glu Arg Pro Trp
65                  70                  75                  80

Pro His Phe Cys Tyr Tyr Trp Thr Lys Glu His Thr His Ser Glu Arg
                85                  90                  95

Pro Trp Pro His Phe Cys Tyr Tyr Trp Arg Lys Ser Ile Arg Ile Gln
            100                 105                 110

Arg Gly Pro Gly Arg Thr Phe Val Thr Ile Gly Lys Ile Arg Lys Ser
        115                 120                 125

Ile Arg Ile Gln Arg Ser Pro Gly Arg Thr Phe Val Thr Ile Ala Lys
    130                 135                 140

Asp Ile Arg Ile Gln Arg Ser Pro Gly Arg Thr Phe Val Thr Ile Gly
145                 150                 155                 160

Lys Lys Thr

<210> 44
<211> 166
<212> PRT
<213> Artificial Sequence

<220>
<223> Description of Artificial Sequence:pTH197

<400> 44
Met Arg Gly Ser His His His His His His Gly Ile Arg Arg Arg Tyr
  1               5                  10                  15

Pro Arg Lys Ser Ile Arg Ile Gln Arg Ala Leu Ala Gly Thr Phe Val
             20                  25                  30

Thr Ile Gly Lys Ser Ile Arg Ile Gln Arg Gly Pro Gly Arg Thr Phe
         35                  40                  45

Val Ile Ile Gly Asn Glu Arg Gly Ile Arg Ile Gln Arg Gly Pro Gly
    50                  55                  60

Arg Thr Phe Val Thr Ile Gly Thr Lys Glu His Thr His Ser Glu Arg
65                  70                  75                  80

Pro Trp Pro His Phe Cys Tyr Tyr Trp Lys Glu His Thr His Ser Glu
                85                  90                  95

Arg Pro Trp Pro His Phe Cys Tyr Tyr Trp Arg Lys Ser Ile Pro Phe
            100                 105                 110

Glu Arg Pro Trp Pro His Phe Cys Tyr Tyr Cys Glu Arg Ala Tyr Ala
        115                 120                 125

Phe Arg Glu Ala Leu Ala Ala Leu Leu Leu Leu Leu Ala Lys Glu His
    130                 135                 140

Thr His Ser Glu Arg Pro Trp Pro His Phe Leu Leu Leu Leu Glu Arg
145                 150                 155                 160

Gly Ser Gly Leu Ile Asn
                165

<210> 45
<211> 94
<212> PRT
<213> Artificial Sequence

<220>
<223> Description of Artificial Sequence:pTH198

<400> 45
Met Arg Gly Ser His His His His His His Gly Ile Arg Arg Arg Tyr
  1               5                  10                  15

Pro Glu Arg Ala Tyr Ala Phe Arg Glu Ala Leu Ala Ala Leu Leu Leu
            20                  25                  30

Leu Leu Ala Lys Glu His Thr His Ser Glu Arg Pro Trp Pro His Phe
        35                  40                  45

Cys Tyr Tyr Trp Thr Lys Glu His Thr His Ser Glu Arg Pro Trp Pro
    50                  55                  60

His Phe Cys Tyr Tyr Trp Lys Asp Arg Lys Ser Ile Arg Ile Gln Arg
 65                  70                  75                  80

Gly Pro Gly Arg Thr Phe Val Thr Ile Gly Gly Asp Leu Gly
                85                  90

<210> 46
<211> 129
<212> PRT
<213> Artificial Sequence

<220>
<223> Description of Artificial Sequence:pTH199

<400> 46
Met Arg Gly Ser His His His His His His Gly Ile Arg Arg Arg Tyr
  1               5                  10                  15

Pro Glu Arg Ala Tyr Ala Phe Arg Glu Ala Leu Ala Ala Leu Leu Leu
             20                  25                  30

Leu Leu Glu Ala Lys Glu His Thr His Ser Glu Arg Pro Trp Pro His
         35                  40                  45

Phe Cys Tyr Tyr Trp Lys Arg Lys Ser Ile Arg Ile Gln Arg Gly Pro
     50                  55                  60

Gly Arg Thr Phe Val Thr Ile Gly Glu Arg Ala Tyr Ala Phe Arg Glu
 65                  70                  75                  80

Ala Leu Ala Ala Leu Leu Leu Leu Leu Glu Arg Arg Lys Ser Ile Arg
                85                  90                  95

Ile Gln Arg Gly Pro Gly Arg Thr Phe Val Tyr Tyr Trp Lys Asp Thr
            100                 105                 110

Lys Glu His Thr His Ser Glu Arg Pro Trp Pro His Phe Trp Asp Leu
        115                 120                 125

Gly

<210> 47
<211> 131
<212> PRT
<213> Artificial Sequence

<220>
<223> Description of Artificial Sequence:pTH200

<400> 47
Met Arg Gly Ser His His His His His His Gly Ile Arg Arg Arg Tyr
1               5                   10                  15

Pro Glu Arg His Thr His Ser Glu Arg Pro Trp Pro His Phe Cys Tyr
            20                  25                  30

Tyr Trp Arg Lys Ser Ile Arg Ile Gln Arg Gly Pro Gly Arg Thr Phe
        35                  40                  45

Val Thr Ile Gly Glu Arg Ala Tyr Ala Phe Arg Glu Ala Leu Ala Ala
    50                  55                  60

Leu Leu Leu Leu Leu Glu Ser Glu Arg Ala Tyr Ala Phe Arg Glu Ala
65                  70                  75                  80

Leu Ala Ala Leu Leu Leu Leu Leu Glu Ser Glu Arg Ala Tyr Ala Phe
                85                  90                  95

Arg Glu Ala Leu Ala Ala Leu Leu Leu Leu Leu Ala Lys Glu His Thr
            100                 105                 110

His Ser Glu Arg Pro Trp Pro His Phe Cys Tyr Tyr Trp Arg Gly Ile
        115                 120                 125

Trp Val Asn
    130


<210> 48
<211> 40
<212> PRT
<213> Artificial Sequence

<220>
<223> Description of Artificial Sequence:Synthesized
      Peptide

<400> 48
Ile Asn Cys Thr Arg Pro Asn Asn Asn Thr Arg Lys Ser Ile Arg Ile
1               5                   10                  15

Gln Arg Gly Pro Gly Arg Thr Phe Asn Thr Ile Gly Lys Ile Gly Asn
            20                  25                  30
Met Arg Gln Ala His Cys Asn Ile
        35                  40